# EUROPEAN PATENT SPECIFICATION

(11) **EP 0 220 689 B1**
(45) Date of publication and mention of the grant of the patent: **15.06.1994**
(21) Application number: 86114769.2
(22) Date of filing: 24.10.1986
(51) Int. Cl.: C12N 15/62, C12N 15/81, C12N 1/14, C12N 1/16, C12N 1/18, C12P 21/02, C07K 7/40

(54) **Method of using bar1 for secreting foreign proteins**
Verfahren zur Verwendung von BAR1 für die Fremdproteinsekretion
Méthode permettant l'utilisation de BAR1 pour la sécrétion de protéines étrangères

(30) Priority: 25.10.1985 US 791305; 20.10.1986 WO PCT/US86/02198
(43) Date of publication of application: 06.05.1987
(73) Proprietor: ZYMOGENETICS, INC., Seattle, WA 98108 (US)
(72) Inventor: MacKay, Vivian L., Seattle, Washington 98105 (US)
(74) Representative: Ebbinghaus, Dieter, Dipl.-Ing.

(56) References cited:
- EP-A- 0 121 884
- DE-A- 3 537 176
- US-A- 4 613 572
- NATURE, vol. 314, 18th April 1985, pages 598-603, London, GB; A.M. MILLER et al.: "Identification and comparison of two sequence elements that confer cell-type specific transcription in yeast"; p. 601

## Description

This is a continuation-in-part of copending Serial No. 791,305, filed october 25, 1985.

The present invention is directed to novel DNA constructs containing at least the translated signal peptide portion of the Saccharomyces cerevisiae BAR1 gene and at least one structural gene foreign to a host cell transformed with said construct. Transformation of host organisms by such constructs will result in expression of a primary translation product comprising the structural protein encoded by the foreign gene fused to the signal peptide of BAR1 so that the protein is processed through the host cell secretory pathway and may be secreted from the host cell into the culture medium or the periplasmic space.

Various procaryotic and eucaryotic microorganisms have been utilized as hosts for production of heterologous polypeptides, i.e., polypeptides which are not naturally produced by the host, by way of recombinant DNA methodology. Various eucaryotic fungal species are of particular interest, including Saccharomyces cerevisiae, Schizosaccharomyces pombe, Aspergillus and Neurospora. In particular, much work has been done in the budding yeast S. cerevisiae. Yeast cells, when transformed with a suitable DNA construct, such as a plasmid, have been made to express heterologous genes contained in the plasmid. However, a major limitation to this technology is that, in many cases, the protein products are not secreted into the medium by the host cells and it is thus necessary to disrupt the cells and purify the desired protein from the various contaminating cellular components without denaturing or inactivating it. Thus, it is desirable to be able to direct the transformed cells to secrete the heterologous product which would simplify purification of that product. Additionally, it may be desirable for some proteins to enter a host cell secretory pathway to facilitate proper processing, i.e., disulfide bond formation.

S. cerevisiae is known to secrete some of its naturally produced proteins, although knowledge of the process is quite limited compared to what is known about secretion of proteins from bacteria and mammalian cells. It appears that most of the secreted yeast proteins are enzymes which remain in the periplasmic space, although the enzymes invertase and acid phosphatase may also be incorporated into the cell wall. The proteins which are known to be secreted into the culture medium by S. cerevisiae include the mating pheromones (α-factor and a-factor), killer toxin, and the protein accounting for Barrier activity (hereinafter "Barrier"). Secretion through the cell wall into the medium is also referred to as "export". S. cerevisiae mating type α cells produce α-factor which is secreted into the culture medium, whereas a cells produce two secreted polypeptides, a-factor and Barrier. The gene for α-factor has been cloned, sequenced, and analyzed (See Kurjan and Herskowitz, Cell 30: 933-943 (1982)). The signal peptide (a short peptide sequence believed to direct the cell to secrete an attached protein), leader sequence (comprising a precursor polypeptide that is cleaved from mature α-factor) and non-translated gene sequences (including promoter and regulatory regions) from the α-factor gene may be used to direct the secretion of foreign proteins produced in yeast. (Brake et al., Proc. Natl. Acad. Sci. USA 81: 4642-4646, 1984) Expression of the α-factor gene is regulated by the MATα1 gene product and processing of the α-factor precursor into the mature protein appears to require at least two steps, believed to be under control of the STE13 and KEX2 genes.

In contrast to α-factor, Barrier appears to be glycosylated, based on its ability to bind concanavalin A. Barrier is produced by a cells and expression appears to be under the control of the MATα2 gene. With the exception of possible signal peptide cleavage, no processing of the Barrier precursor has heretofore been demonstrated and the STE13 and KEX2 genes are not believed to be involved in the expression of Barrier.

Due to the above differences between the control of expression and processing of α-factor and Barrier, one cannot determine a priori which of these yeast genes is better suited to direct the secretion of a particular foreign protein. Because the two proteins appear to be processed through different secretory pathways, it would be desirable to exploit the characteristics of the Barrier secretion system.

It is therefore an object of the present invention to provide DNA constructs containing a segment of the BAR1 gene which codes for at least the signal peptide and also containing a foreign structural gene which results in expression in a microbial host of a heterologous protein which is processed through the secretory pathway of the host cell.

It is a further object of the present invention to provide a method for expressing foreign genes in a microbial host which results in a heterologous protein or a portion thereof encoded by such a gene being processed through the secretory pathway of the host cell.

Another object of the present invention is to provide a method for producing foreign proteins which are secreted from a microbial host.

It is yet a further object of the present invention to provide a method for producing foreign proteins by recombinant DNA technology.

It is another object to provide a method for producing proteins having the amino acid sequences of human proinsulin and insulin by recombinant DNA technology.

These and other objects will become apparent from the following description of the specific embodiments and claims.

The present invention is directed to DNA constructs and methods for using same, which constructs comprise at least the signal peptide coding sequence of the Saccharomyces cerevisiae BAR1 gene, at least one structural gene foreign to the host organism, and a promoter which controls the expression in a host organism of a fusion protein comprising the BAR1 signal peptide and the foreign protein.

In the accompanying drawings:
FIG. 1 shows the nucleotide sequence of the BAR1 gene and the derived amino acid sequence of the primary translation product. The MATα2 binding site is underlined, the putative signal peptide cleavage site is indicated with an arrow, and potential glycosylation sites are marked with asterisks.
FIG. 2 is a diagram of the plasmid pZV9;
FIG. 3 illustrates the construction of plasmid p254;
FIG. 4 illustrates the construction of the plasmids pZV30, pZV31, pZV32 and pZV33;
FIGS. 5A and 5B illustrate the construction of the plasmid pZV50;
FIG. 6 illustrates the construction of the plasmid m115;
FIG. 7 illustrates the construction of the plasmid pZV49;
FIG. 8 illustrates the construction of the plasmid pZV134 comprising the TPI1 promoter;
FIG. 9 illustrates the subcloning of a portion of the MFα1 gene;
FIG. 10 illustrates the construction of plasmid pZV75;
FIG. 11 illustrates the construction of plasmids comprising the TPI1 promoter and BAR1-MFα1 fusions;
FIG. 12 illustrates the construction of plasmid pSW22;
FIG. 13 illustrates the construction of plasmids comprising BAR1-MFα1 fusions;
FIG. 14 illustrates the construction of pZV100;
FIG. 15 illustrates the construction of plasmid pZV102;
FIG. 16 illustrates the construction of the plasmid pSW96;
FIG. 17 illustrates the construction of the plasmid pSW97; and
FIG. 18 illustrates the construction of plasmids pSW98 and pSW99. Δ indicates the mutation at codon 25.

As used herein the term "DNA construct" means any DNA molecule, including a plasmid, which has been modified by human intervention in a manner such that the nucleotide sequences in the molecule are not identical to a sequence which is produced naturally. The term "DNA construct" also includes clones of DNA molecules which have been so modified. The terms "expression vector" and "expression plasmid" are defined as a DNA construct which includes a site of transcription initiation and at least one structural gene coding for a protein of interest which is to be expressed in a host organism. An expression vector will usually also contain appropriate regions such as a promoter and terminator which direct the expression of the protein of interest in the host organism and on origin of replication. Expression vectors according to the present invention will also usually contain a selectable marker, such as a gene for antibiotic resistance or a nutritional marker.

The term "DNA construct" will also be considered to include portions of the expression vector integrated into the host chromosome.

The term "plasmid" will have its commonly accepted meaning, i.e., an autonomously replicating DNA construct, usually close-looped.

The term "signal peptide" refers to that portion of a primary translation product which directs that product into the secretory pathway of the cell which produces it. The signal peptide is usually cleaved from the remainder of the nascent polypeptide by a signal peptidase during this process. A signal peptide is characterized by the presence of a core of hydrophobic amino acids, occurs at the amino terminus of the primary translation product, and is generally from about 17 to 25 amino acides in length. Signal peptidase cleavage sites have been characterized by von Heinje (Eur. J. Biochem. 133:17, 1983). As used herein, the term "signal peptide" may also refer to functional portions of the naturally occurring signal peptide.

The present invention provides a method for directing transformed cells to direct heterologous proteins through a secretory pathway accomplished by transforming a host with a DNA construct containing a foreign gene linked to the yeast BAR1 gene or a portion thereof which codes at least for the BAR1 signal peptide. The proteins so processed may be secreted into the periplasmic space or the culture medium. The yeast BAR1 gene codes for Barrier activity which is believed to be a glycosylated protein secreted by S. cerevisiae a cells. The secreted Barrier allows mating type a cells to overcome the G1 arrest induced by α-factor. It is believed that Barrier may be a protease. (See Manney, J. Bacteriol., 155: 291-301, 1983). Transcription of the BAR1 gene is stimulated by α-factor, Barrier, or an analogous activity, is not detected in α or a/α cells and the BAR1 gene is not transcribed in these cell types.

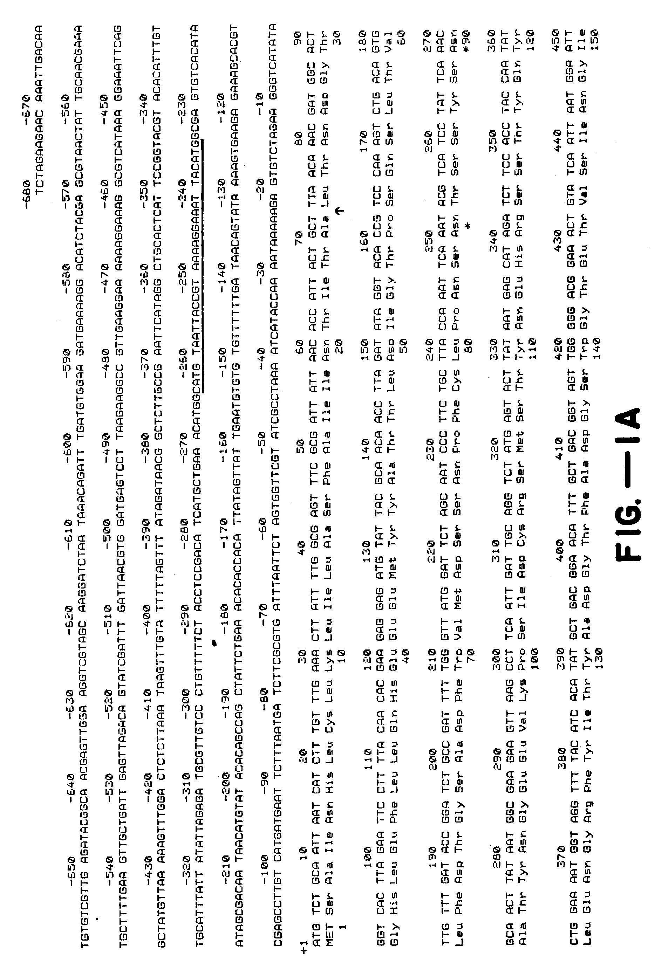

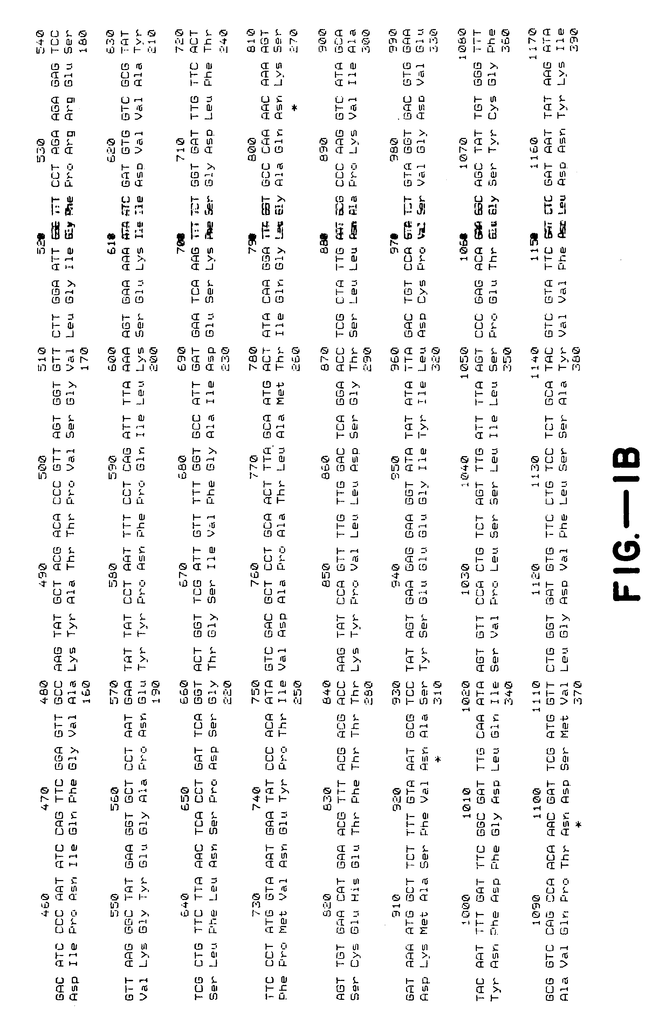

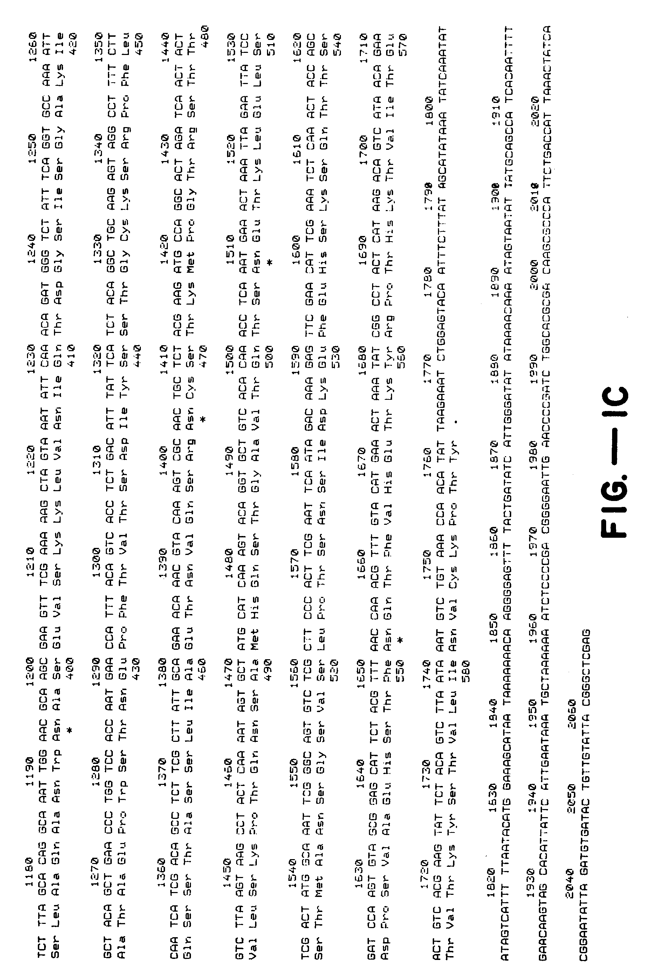

The sequence of 2750 base pairs encompassing the BAR1 gene is shown in Figure 1, together with the derived amino acid sequence of the primary translation product. The ATG translation initiation site of BAR1 is at position 681 of the approximately 2.75 kb fragment shown in Figure 1 which was subcloned from a fragment obtained from a yeast genomic library (Nasmyth and Tatchell, Cell 19: 753-764, 1980). An open reading frame starts with the ATG codon at +1 and extends 1761 bp in the 3' direction. The sequence of the first 24 amino acids of the BAR1 primary translation product appears to be similar to sequences of known yeast and mammalian signal peptides. Thus, the alanine at position 24 may be used as a cleavage site, as in yeast invertase and acid phosphatase. Cleavage could also occur after amino acid 23. At least nine potential asparagine-linked glycosylation sites exist in the primary translation product, although the extent of glycosylation of the mature secreted Barrier is not yet known. The promoter and regulatory regions of the BAR1 gene are located within a region of approximately 680 bp on the 5' side of the translation initiation codon. Full promoter function and response to α-factor stimulation have been localized to the ATG-proximal approximately 680 bp of the 5' untranslated region.

The DNA constructs of the present invention will preferably encode a cleavage site at the junction of the Barrier and foreign protein portions. A preferred such site is a KEX2 cleavage site, a sequence of amino acids which is recognized and cleaved by the product of the S. cerevisiae KEX2 gene (Julius et al. Cell 37: 1075-1080, 1984). A KEX2 site is characterized by a pair of basic amino acids, such as lysine and arginine. It is preferred that the sequence of the KEX2 site be Lys-Arg or Arg-Arg. The BAR1 primary translation product contains two such pairs located in the structural region: Arg-Arg at positions 177-178 and Lys-Lys at positions 404-405. As noted above, the KEX2 gene is not involved in the processing of the Barrier precursor protein, suggesting that the potential processing sites are blocked by protein conformation or glycosylation and further suggesting that Barrier may normally be processed through a pathway different than that used by such KEX2-processed proteins as α-factor. However, applicants have found that by including a KEX2 processing site in a fusion protein comprising a portion of the BAR1 gene primary translation product comprising the signal peptide thereof, together with a protein of interest, the fusion protein is cleaved at the KEX2 site, resulting in secretion of the protein of interest. It has also been found that by reducing the efficiency of signal peptidase cleavage of the Barrier portion of a fusion protein comprising a KEX2 cleavage site, enhanced levels of the protein of interest are exported. The KEX2 cleavage site may be provided by the BAR1 sequence or the gene of interest, or may be introduced into the fusion by linker addition, site-specific mutagenesis, etc.

Thus, according to the present invention, a portion of the BAR1 gene comprising the ATG initiation codon and signal peptide coding sequence thereof may be joined to a foreign gene of interest and transformed into a eukaryotic host cell. The resultant fusion gene will include a processing site, preferably a KEX2 cleavage site, at the junction of the BAR1 and foreign sequences. Such a construct may also comprise regulatory regions and the promoter from the 5' non-coding region of the BAR1 gene, or may comprise regulatory regions and/or promoters from other genes. In addition to the promoter from the BAR1 gene, other promoters which may be used include the promoters from the S. cerevisiae alcohol dehydrogenase I or alcohol dehydrogenase II genes, the genes of the S. cerevisiae glycolytic pathway, such as the TPI1 promoter, and corresponding genes from other species, including the fission yeast Schizosaccharomyces pombe (Russell and Hall, J. Biol. Chem. 258: 143-149, 1983 and Russell, Nature 301: 167-169, 1983). The S. cerevisiae alcohol dehydrogenase I gene has been described by Ammerer (Methods in Enzymology 101: 192-201, 1983). The alcohol dehydrogenase II gene has been described by Russell et al. (J. Biol. Chem. 258: 2674-2682, 1983). Glycolytic genes of S. cerevisiae have been described by Kawasaki (Ph. D. Thesis, University of Washington, 1979), Hitzeman et al. (J. Biol. Chem. 225: 12073-12080), 1980), Kawasaki and Fraenkel (Biochem. Biophys. Res. Comm. 108: 1107-1112, 1982) and Alber and Kawasaki (J. Mol. Appl. Genet. 1: 419-434, 1982).

In a preferred embodiment, the signal peptide coding sequence of the BAR1 gene is altered to reduce the efficiency of signal peptidase cleavage of the Barrier portion of a fusion protein comprising a KEX2 cleavage site. This may be accomplished by site-specific mutagenesis of potential cleavage sites, preferably those sites at the amino acid 23-24 (of the Barrier protein sequence) juncture or at the amino acid 24-25 juncture.

Methods used to form DNA constructs according to the present invention involve conventional techniques. The structural BAR1 gene, or a portion thereof, and the structural gene to be expressed will preferably be under control of a single promoter. Methods of ligation of DNA fragments are amply described and are well within the ability of those with ordinary skill in the art to perform. The DNA coding sequence of the protein to be expressed may be essentially that of any protein, particularly proteins of commercial importance, such as interferons, insulin, proinsulin, α-1-antitrypsin, growth factors, and tissue plasminogen activator.

After preparation of the DNA construct containing the BAR1 gene or a portion thereof and the structural gene to be expressed, the construct will be transformed into the host organism under transforming conditions. Techniques for transforming prokaryotes and eukaryotes (including mammalian cells) are known in the literature.

Preferably, the host organism will be a strain of the budding yeast Saccharomyces cerevisiae; however, other fungi, including the fission yeast Schizosaccharomyces pombe and the filamentous fungi Aspergillus nidulans and Neurospora spp. may also be used.

The following examples are given by way of example and not by way of limitation. Unless otherwise indicated, standard molecular biology methods were used throughout. Restriction endonucleases were obtained from Bethesda Research Laboratories, New England BioLabs and Boehringer-Mannheim Biochemicals and were used as directed by the manufacturer, generally with the addition of pancreatic RNase (10 »g/ml) to digests. T4 DNA ligase was obtained from Bethesda Research Laboratories or Boehringer-Mannheim and was used as directed. M13 and pUC host strains and vectors were obtained from Bethesda Research Laboratories. M13 cloning was done as described by Messing, Methods in Enzymology 101, 20-77 (1983). DNA polymerase I (Klenow fragment) was used as described in Maniatis et al., Molecular Cloning: A Laboratory Manual, Cold Spring Harbor Laboratory (1982). E. coli cultures were transformed by the method of Bolivar et al., Gene 2, 95-113 (1977). S. cerevisiae cultures were transformed by the method of Beggs, Nature 275, 104-108 (1978) as modified by MacKay, Methods in Enzymology 101, 325 (1983). S. pombe was transformed as described by Russell, Nature 301, 167-169 (1983). The mating pheromone α-factor was prepared by the method of Duntze et al., Eur. J. Biochem. 35: 357-365, 1973, as modified by Manney et al., J. Cell Biol. 96: 1592-1600, 1983 or was purchased from Sigma Chemical Co. Oligonucleotides were synthesized on an Applied Biosystems Model 380A DNA synthesizer and purified by polyacrylamide gel electrophoresis on denaturing gels.

### METHODS

### Assay for Barrier Activity

The assay used for detection of Barrier production by transformed yeast cells relies on the ability of Barrier to reverse the inhibition of growth of sensitive a cells exposed to α-factor. The test strain is one that is abnormally sensitive to α-factor, such as strain RC629 (MATa bar1), since it produces no Barrier activity. A lawn is prepared using such a strain in a soft agar overlay on an agar plate. A sufficient quantity of α-factor (0.05 - 0.1 unit, as assayed by Manney, ibid.) is added to the overlay to inhibit growth of the cells. Transformants to be screened for Barrier production are then spotted onto the lawn. Secretion of Barrier by the transformed cells reverses the α-factor growth inhibition immediately surrounding the spot, thereby allowing the sensitive cells to recover. The recovered cells are observed as a fringe of growth around the normally smooth edge of the colony of transformed cells. The presence of this fringe indicates that the plasmid in the transformed strain directs the expression and secretion of Barrier.

### IRI and IRC Assays

IRI and IRC assays were performed using commercial kits obtained from Novo Industri, Bagsvaerd, Denmark. Guinea pig anti-porcine insulin and guinea pig anti-human C-peptide antibodies are supplied with the kits.

### ASSAY FOR IRI

50 »l sample in NaFAM (0.04 M phosphate buffer pH 7.4 containing bovine serum albumin)
50 »l antibody (stock diluted 1:30)
16-24 hours at 4° C
50 »l ¹²⁵I-insulin (diluted 1:100)
2 hours at 4° C
50 »l 1% Staphylococcus aureus in NaFAM
45 min. at 0° C
Wash twice with 1% BSA/TNEN*
Centrifuge and count pellets
The different steps can be conveniently done in microtiter dishes, until the pellets are transferred to the scintillation vials.

### ASSAY FOR IRC

50 »l sample in NaFAM
50 »l antibody (stock diluted 1:50)
16-24 hours at 4° C
50 »l ¹²⁵I-C peptide (stock diluted 1:30)
2-4 hours at 4° C
50 »l 1% S. aureus in NaFAM
45 min. 0° C
Wash twice 1% BSA/TNEN*
Centrifuge and count pellets
*TNEN is 20 mM Tris pH 8.0
100 mM NaCl
1 mM EDTA
0.5% NP-40

### ASSAY FOR ALPHA-FACTOR ACTIVITY

The assay used for detection of α-factor export by transformed yeast cells employs the ability of α-factor to inhibit the growth of sensitive a cells. The test strain (such as S. cerevisiae strain RC629 (MATa bar1)) contains a mutation in the BAR1 gene that prevents the production of Barrier activity and renders the cells super-sensitive to α-factor. A lawn of the test strain is made in a soft agar overlay over a plate of standard yeast selective synthetic medium (e.g. medium lacking leucine). Transformants to be screened for α-factor export are spotted onto the lawn and incubated at 30°C. Secretion of α-factor by the transformants will cause growth inhibition in the lawn immediately surrounding the colony. The halo of growth inhibition in the lawn of test cells indicates that the colony is exporting active α-factor. A comparison of halo size enables one to estimate the relative quantities of α-factor exported by each transformant.

### EXAMPLE 1

### Expression of Proinsulin in S. cerevisiae Using The BAR1 Gene

A recombinant plasmid pool comprising the entire yeast genome was constructed (Nasmyth and Tatchell, Cell 19: 753-764, 1980) using the shuttle vector YEp13 (Broach et al., Gene 8: 121-133, 1979). Yeast DNA fragments, produced via a partial Sau 3A digestion, were inserted into Bam HI-digested YEp13. The plasmid pool was used to transform S. cerevisiae strain XP635-10C (MATa leu2-3 leu2-112 bar1^{..}1 gal2: ATCC #20679) and transformants were selected for leucine prototrophy and growth on a concentration of α-factor that is inhibitory to the a bar1 cells. Resultant colonies were then screened for the ability to secrete Barrier activity. Two colonies were found which carried both leucine independence and the ability to secrete Barrier. These colonies carried the plasmids designated pBAR2 and pBAR3.

Plasmid DNA isolated from those two transformants was used to transform E. coli strain RRI (ATCC #31343). Transformants were selected for ampicillin resistance. Plasmids pBAR2 and pBAR3 were purified from the E. coli transformants and characterized by restriction endonuclease digestion and electrophoresis on agarose or acrylamide gels. Plasmid pBAR2 was shown to contain an insert of approximately 9.2 kilobases. E. coli RRI transformed with plasmid pBAR2 has been deposited with ATCC under accession number 39410.

Subcloning showed that the pBAR3 plasmid insert comprised a portion of the insert of pBAR2, but oriented in the vector in the opposite direction. Further subcloning and screening for Barrier secretion localized the functional BAR1 gene sequence to a region of approximately 2.75 kb. This fragment comprises the coding sequence, nontranslated transcribed sequences, promoter, regulatory regions, transcription terminator, and flanking chromosomal sequences.

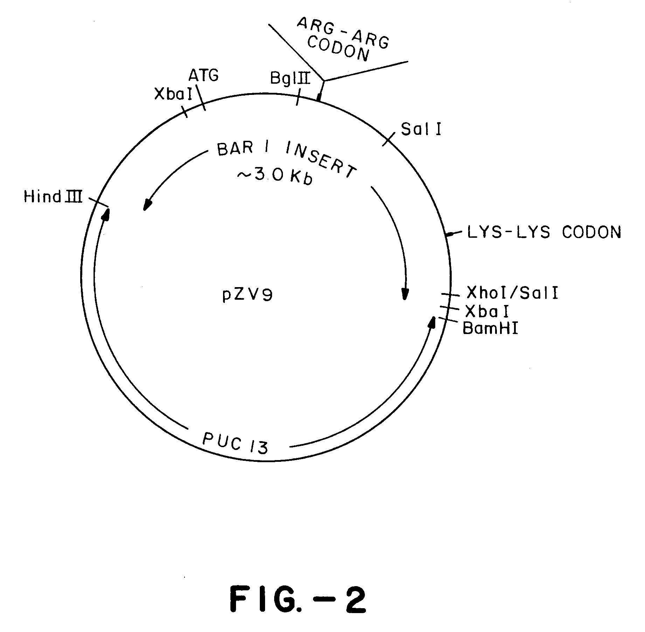

The plasmid pBAR2 was digested with restriction endonucleases Hind III and Xho I and a fragment of approximately 3 kb was purified by electrophoresis through an agarose gel. This fragment was inserted into plasmid pUC13 which had been digested with Hind III and Sal I. The resulting recombinant plasmid, designated pZV9 (FIG. 2), can be used to transform E. coli, but lacks the necessary origin of replication and selectable marker for a yeast vector. The plasmid pZV9, in a transformant strain of E. coli RRI has been deposited with ATCC under accession no. 53283.

For the BAR1 gene to be used to direct the secretion of proinsulin, fragments of the BAR1 gene comprising the 5' regulatory region and a portion of the coding sequence were used. The fusion of the BAR1 and proinsulin gene fragments was made in the proper reading frame and at a point in the BAR1 sequence at which the resulting fusion polypeptide may be cleaved, preferably in vivo. Several sites in the BAR1 gene are potential cleavage sites; the Arg-Arg at position 177-178 was selected as the test site of fusion with proinsulin. Accordingly, the 5' regulatory sequences and approximately 800 bp of the coding sequence of BAR1 were purified from plasmid pZV9 as a 1.9 kb Hind III-Sal I fragment.

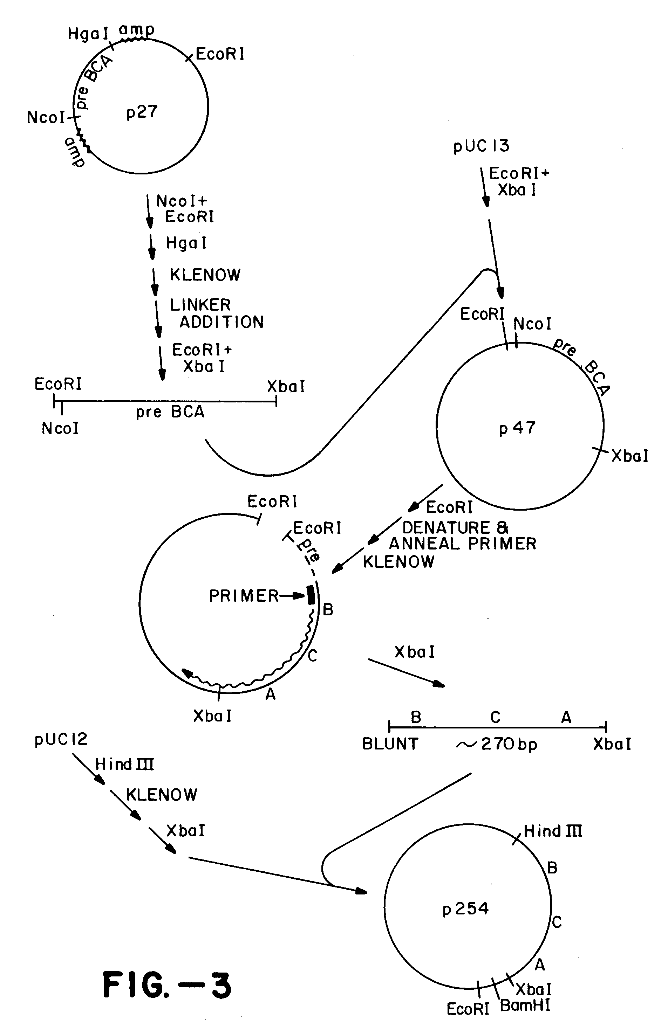

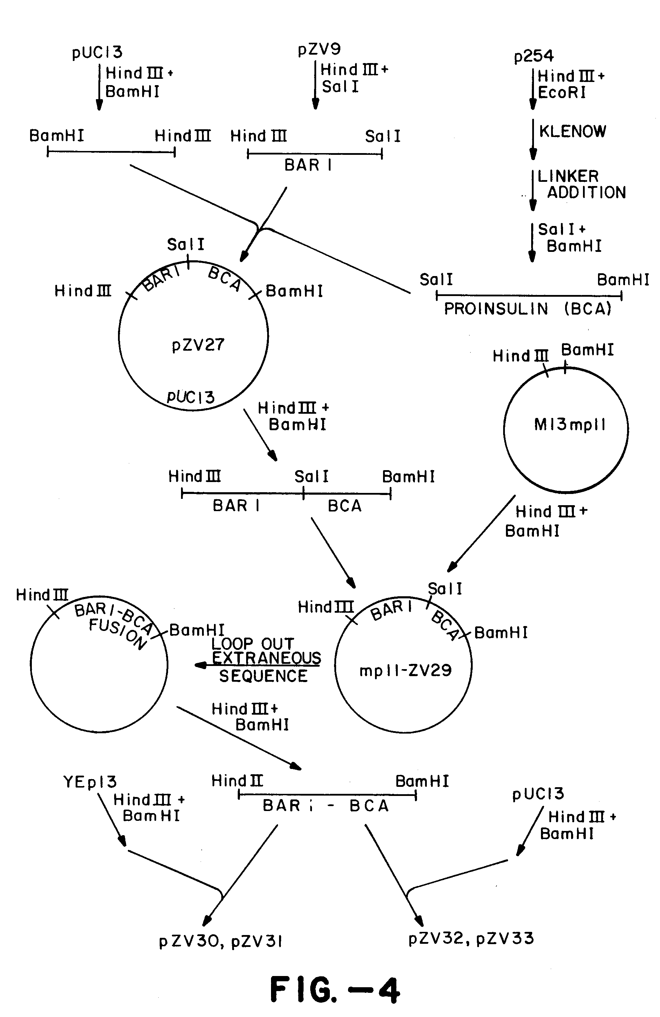

Referring to FIG. 3, there is shown a method for subcloning human preproinsulin cDNA. A human preproinsulin cDNA (preBCA clone), p27, is produced by G-tailing Pst I digested pBR327 and inserting C-tailed DNA, made by reverse transcribing total RNA from human pancreas. Plasmid pBR327 is described by Soberon et al., Gene 9: 287-305 (1980) and the sequence of human preproinsulin is reported by Bell et al., Nature 232: 525-527 (1979). The complete translated sequence was cut out as a Nco I-Hga I fragment. The protruding ends were filled in with DNA-polymerase I (Klenow fragment), and synthetic Eco RI linkers (GGAATTCC) and Xba I linkers (CTCTAGAG) were attached simultaneously. The fragment was subcloned into pUC13 (Vieira and Messing, Gene 19: 259-268, 1982; and Messing, Meth. in Enzymology 101: 20-77, 1983) which had been cut with Eco RI and Xba I. Because the addition of an Eco RI linker at the 5' end restores the Nco I site (CCATGG) at the initiation codon, plasmids were screened for the presence of Eco RI, Nco I and Xba I sites flanking a 340 bp insert. A plasmid having these properties is termed p47, shown in FIG. 3. A proinsulin (BCA) fragment with a blunt 5' end was generated by primer repair synthesis (Lawn et al., Nuc. Acids Res. 9: 6103-6114, 1981) of plasmid p47. Subsequent digestions with Xba I yielded a 270 bp fragment which was inserted into pUC12. (Vieira and Messing, ibid., and Messing, ibid). The vector was prepared by cutting with Hind III, blunting the ends with DNA polymerase I (Klenow fragment), cutting with Xba I, and gel purifying. The resultant vector fragment, comprising a blunt end and a Xba I sticky end, was ligated to the above described BCA fragment. As mature BCA starts with the amino acid phenylalanine (codon TTT), blunt end ligation of the two fragments regenerates a Hind III site at the junction. Plasmids were screened first for the recovery of the Hind III site and then by sequencing across the junction using an M13 sequencing primer. Plasmid p254 had the correct sequence.

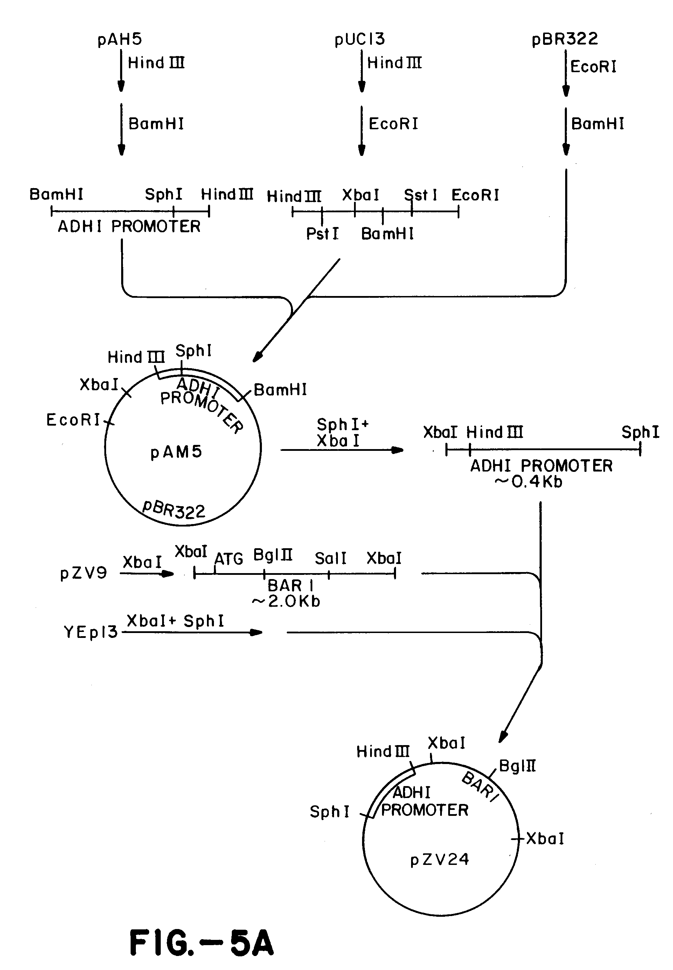

Referring to FIG. 4, plasmid p254 was digested with Hind III and Eco RI and the ca. 270 bp proinsulin fragment was gel purified. The fragment ends were blunted using DNA polymerase I (Klenow fragment) and deoxynucleotide triphosphates. Sal I linker sequences (GGTCGACC) were treated with T4 polynucleotide kinase and γ-³²P-ATP and were ligated to the blunted proinsulin fragment. Digestion with Sal I and Bam HI, followed by electrophoresis on a 1.5% agarose gel, yielded a proinsulin fragment with Sal I and Bam HI cohesive ends.

The proinsulin fragment and the 1.9 kb BAR1 fragment were ligated together into pUC13 which had been digested with Hind III and Bam HI. This construct was used to transform E. coli K12 (JM83).

Transformed cells were screened for ampicillin resistance and production of white colonies. Further screening by restriction endonuclease digestion using Hind III, Bam HI, and Sal I identified a plasmid (pZV27) containing a Hind III-Bam HI fragment of the proper size and a single Sal I site.

In order to link the first amino acid of proinsulin to the Arg-Arg potential processing site of the BAR1 gene product, the intervening material in the BAR1-proinsulin fusion was deleted. A synthetic oligonucleotide was used to direct the looping-out of this extraneous material in the following manner. Referring to FIG. 4, plasmid pZV27 was digested with Hind III and Bam HI and the ca. 2.2 kb BAR1-proinsulin fusion fragment was gel purified. This fragment was then inserted into the replicative form of the phage vector M13mp11 (Messing, Meth. in Enzymology 101: 20-77, 1983) which had been digested with Hind III and Bam HI. This recombinant DNA was used to transfect E. coli K12 (JM103) (Messing, ibid.). White plaques were picked and the replicative forms of the recombinant phage were screened for the correct restriction patterns by double enzyme digestions using Hind III + Sal I and Sal I + Bam HI. A construct showing the desired pattern is known as mp11-ZV29. The oligonucleotide primer (sequence: 3' GGATCTTCTAAACACTTG 5') was labelled using γ-³²P-ATP and T4 polynucleotide kinase. 7.5 pmol of kinased primer was then combined with 80 ng of M13 sequencing primer (Bethesda Research Laboratories, Inc.) This mixture was annealed to 2 »g of single stranded mp11-ZV29 and the second strand was extended using T4 DNA ligase and DNA polymerase I (Klenow fragment), as described for oligonucleotide-directed mutagenesis (two primer method) by Zoller et al. (Manual for Advanced Techniques in Molecular Cloning Course, Cold Spring Harbor Laboratory, 1983). DNA prepared in this way was used to transfect E. coli K12 (JM103) and plaques were screened using the kinased oligomer as probe (Zoller et al., ibid.). Plaques so identified were used for preparation of phage replicative form (RF) DNA (Messing, ibid.). Restriction enzyme digestion of RF DNA identified two clones having the proper Xba I restriction pattern (fragments of 7.5 kb, 0.81 kb, and 0.65 kb) and lacking a Sal I restriction site (which was present in the deleted region of the BAR1-proinsulin fusion).

RF DNA from these two clones was digested with Hind III and Bam HI, and the 1.9 kb fusion fragment from each was gel purified. These fragments were ligated to pUC13 and YEp13 (Broach et al., Gene 8:121-133, 1979) vectors which had been digested with Hind III and Bam HI. pUC/BAR1-proinsulin hybrid plasmids for subsequent sequencing were used to transform E. coli K12 (JM83). Two of these plasmids were designated pZV32 and pZV33. YEp13-derived recombinants were used to transform E. coli RR1 (Nasmyth and Reed, Proc. Nat. Acad. Sci. USA 77:2119-2123, 1980). Two of these plasmids were designated pZV30 and pZV31 (Fig 4).

Sequencing of pZV32 and pZV33 was done by the method of Maxam and Gilbert (Meth. in Enzymology 65:57, 1980). The BAR1-proinsulin fusion was sequenced from the Bgl II site located approximately 190 bp to the 5' side of the junction and from the Sau 96I site located about 140 bp to the 3' side of the junction (in the proinsulin gene). Data from these experiments confirmed that the desired fusion between the BAR1 and proinsulin genes had been constructed.

S. cerevisiae strain XP635-10C was transformed with plasmids pZV30 and pZV31. One liter cultures were grown in standard yeast synthetic medium lacking leucine. After 34 hrs., α-factor was added to a 10 ml aliquot of each culture. After an additional 11 hours, cultures were harvested by centrifugation. Cell pellets and supernatants were tested for the presence of insulin or insulin-like material. Results of two such assays on the supernatant of a culture transformed with plasmid pZV31 showed 3 pmole IRI material per ml of culture medium, and 5.8 pmol IRC material per ml of culture medium. IRI is correctly folded insulin, proinsulin, or degradation products thereof. IRC is free C-peptide, incorrectly folded proinsulin, or degradation products thereof.

### EXAMPLE 2

### Expression of Proinsulin in S. cerevisiae Using The Alcohol Dehydrogenase I Promoter, BAR1 Gene, and Triose Phosphate Isomerase Terminator

The S. cerevisiae alcohol dehydrogenase I promoter (hereinafter ADHI promoter; also known as ADCI promoter) was tested for use in directing expression of foreign polypeptides in conjunction with BAR1 sequences. A plasmid comprising these sequences was constructed.

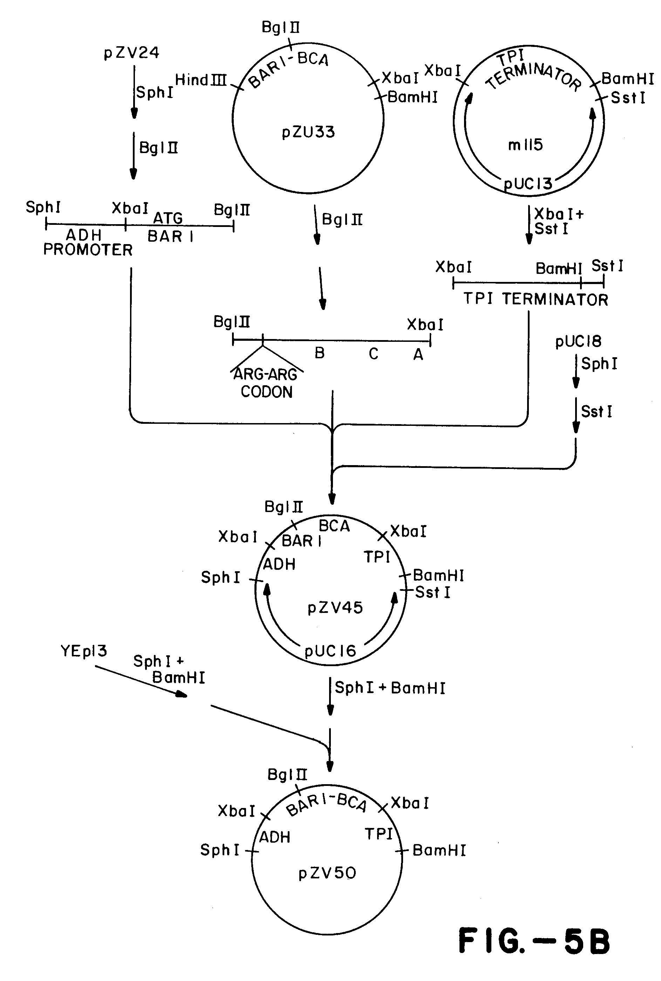

The plasmid pZV50 (FIG. 5B) comprises the S. cerevisiae ADHI promoter, the BAR1-proinsulin fusion described above, and the terminator region of the S. cerevisiae triose phosphate isomerase (TPI1) gene (Alber and Kawasaki, J. Molec. Appl. Genet. 1: 419-434, 1982). It was constructed in the following manner. Referring to FIG. 5A, plasmid pAH5 (Ammerer, ibid.) was digested with Hind III and Bam HI and the 1.5 kb ADHI promoter fragment was gel purified. This fragment, together with the Hind III-Eco RI polylinker fragment from pUC13, was inserted into Eco RI, Bam HI-digested pBR327, using T4 DNA ligase. The resultant plasmid, designated pAM5, was digested with Sph I and Xba I, and the approximately 0.4 kb ADHI promoter fragment was purified on a 2% agarose gel. Plasmid pZV9 was digested with Xba I, and the approximately 2 kb BAR1 fragment, containing the entire BAR1 coding region, was similarly gel purified. These two fragments, ADHI promoter and BAR1 sequence, were ligated to Xba I, Sph I-digested YEp13 to produce plasmid pZV24. Digestion of pZV24 with Sph I and Bgl II, followed by gel purification, yielded an ADHI promoter-BAR1 fusion of approximately 800 bp, which contains the ATG translation start codon, but lacks the codons for the Arg-Arg potential processing site. Plasmid pZV33, containing the BAR1-proinsulin fusion, was digested with Bgl II and Xba I, and the fusion fragment (ca. 500 bp), which includes the Arg-Arg codons, was purified.

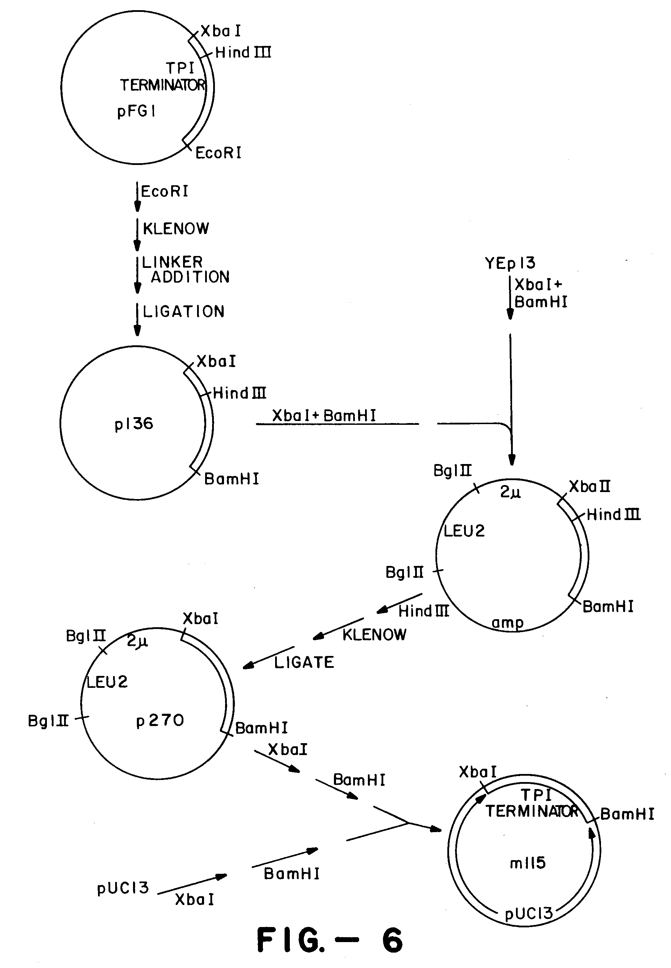

Referring to FIG. 6, The TPI1 terminator was obtained from plasmid pFG1 (Alber and Kawasaki, ibid.). pFG1 was digested with Eco RI, the linearized plasmid ends were blunted using DNA polymerase I (Klenow fragment), and Bam HI linker sequences (CGGATCCA) were added. The fragment was digested with Bam HI and religated to produce plasmid p136. The 700 bp TPI1 terminator was purified from p136 as a Xba I-Bam HI fragment. This fragment was inserted into Xba I, Bam Hl-digested YEp13, which was then cut with Hind III, the ends blunted using DNA polymerase I (Klenow fragment), and religated to produce plasmid p270. The TPI1 terminator was purified from p270 as a Xba I-Bam HI fragment, and was inserted into Xba I, Bam HI-digested pUC13 to yield plasmid m115.

Referring to Figure 5B, the TPI1 terminator was removed from plasmid m115 by digestion with Xba I and Sst I, followed by gel purification. The three fragments: ADHI-BAR1 fusion, BAR1-proinsulin fusion, and TPI1 terminator, were inserted into plasmid pUC18 (Norrander et al., Gene 26: 101-106, 1983) which had been digested with Sph I and Sst I. This DNA was used to transform E. coli K12 (JM83). Selection for ampicillin resistance and screening for production of white colonies identified a plasmid (pZV45) containing the desired inserts. Plasmid pZV45 was subsequently digested with Sph I and Bam HI, and the ADHI-BAR1-proinsulin-TPI terminator sequence was gel purified. This fragment was inserted into YEp13 which had been digested with Sph I and Bam HI, to produce the S. cerevisiae expression vector pZV50.

S. cerevisiae strain XP635-10C was transformed with plasmid pZV50 and cultured and assayed as described in Example 1 above. No IRI material was found in the medium, and IRC material was less than 0.5 pmol per ml. Cells extracted with 0.1% Nonidet P-40 showed 1 pmol IRC material per ml of cell extract.

### EXAMPLE 3

### Expression of Proinsulin in Schizosaccharomyces pombe Using the BAR1 Gene and S. pombe Alcohol Dehydrogenase Promoter

This example demonstrates the use of portions of the BAR1 gene to direct the secretion of foreign polypeptides expressed in a transformed Schizosaccharomyces pombe host. A plasmid was constructed which combines the S. pombe alcohol dehydrogenase (ADH) gene promoter with the BAR1-proinsulin gene fusion.

The S. pombe ADH promoter was obtained from a library of DNA fragments derived from S. pombe strain 972h⁻ (ATCC 24843), which had been cloned into YEp13 as described by Russell and Hall (J. Biol. Chem. 258: 143-149, 1983). The promoter sequence was purified from the library as a 0.75 kb Sph I-Eco RI fragment. This fragment and the Eco RI-Hind III polylinker fragment of pUC12 were ligated into YEp13 which had been digested with Sph I and Hind III. The resulting plasmid is known as pEVP-11.

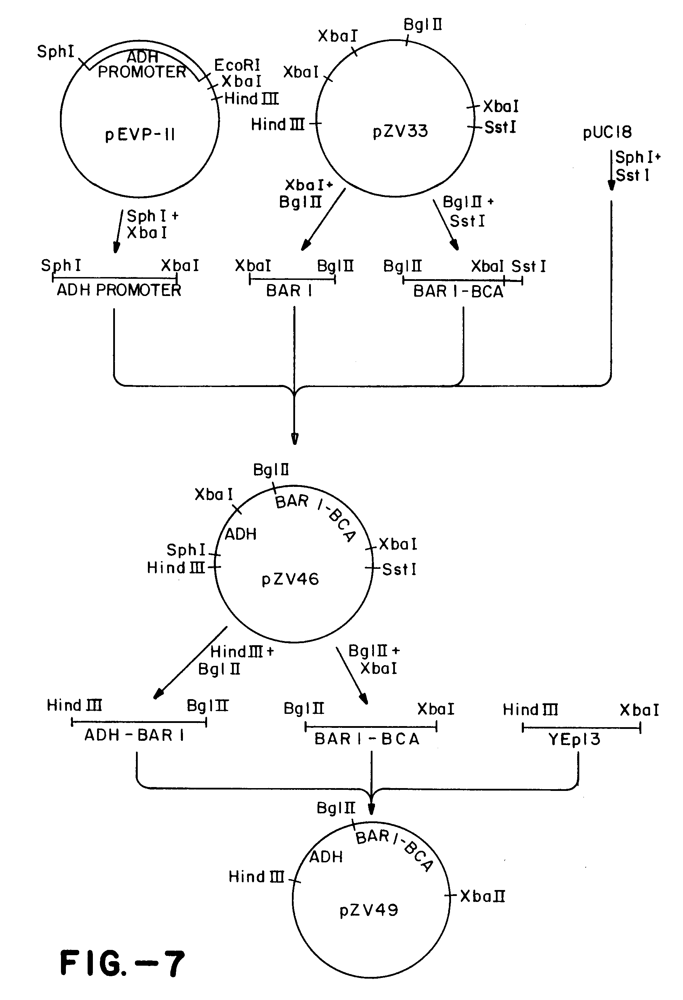

Referring to FIG. 7 for constructing the S. pombe expression vector, the ADH promoter was purified from pEVP-11 as a Sph I-Xba I fragment. Plasmid pZV33 was digested with Xba I and Bgl II and the ca. 340 bp BAR1 fragment, which includes the ATG initiation codon, was purified. pZV33 was digested with Bgl II and Sst I, and the BAR1-proinsulin fusion sequence was purified. The three fragments were combined with Sph I, Sst I-digested pUC18 to produce plasmid pZV46. As pUC18 is not effective for transformation of S. pombe, the plasmid was subjected to two double enzyme digestions. An ADH promoter-BAR1 fusion fragment was purified from a Hind III + Bgl II digest, and a BAR1-proinsulin fusion sequence was purified from a Bgl II + Xba I digest. These fragments were inserted into Hind III, Xba I-digested YEp13 to produce S. pombe expression vector pZV49.

One liter cultures of transformed S. pombe strain 118-4h⁻ (ATCC #20680) were grown 36 hours at 30°C in standard yeast synthetic medium (-leu D) containing 200 mg/l aspartic acid and 100 mg/l each of histidine, adenine, and uracil. Cultures were harvested by centrifugation and the supernatants assayed by IRI and IRC assays. Two samples from pZV49-transformed cells contained 1.6 pmol/ml IRI material and 0.5 pmol/ml IRC-reactive material, respectively. A control sample from a culture transformed with YEp13 contained no detectable IRC-reactive material.

### EXAMPLE 4

### Export Of Alpha-Factor Using BAR1 Signal Peptide

The BAR1 signal peptide was tested for its ability to direct the export of α-factor from a yeast transformant. Several plasmids containing DNA fragments coding for fusion proteins with varying lengths of the BAR1 protein and 1 or 4 copies of mature α-factor were constructed. These plasmids were transformed into an a/α diploid host strain and the transformants assayed for α-factor production by the halo assay.

Plasmids pSW94, pSW95, pSW96, and pSW97 comprise the S. cerevisiae triose phosphate isomerase (TPI1) promoter, a 355 bp or 767 bp fragment of the BAR1 gene (comprising 114 or 251 codons of the 5' end of the BAR1 coding sequence, respectively) and either one or four copies of the alpha-factor (MFα1) coding sequence. These constructs are described in Table 1.

**TABLE 1**

| Plasmid | BAR1 Fragment | α-factor |
|---|---|---|
| pSW94 | 355 bp | 4 copies |
| pSW95 | 767 bp | 4 copies |
| pSW96 | 355 bp | 1 copy |
| pSW97 | 767 bp | 1 copy |

Plasmid pM220 (also known as pM210) was used as the source of the TPI1 promoter fragment. E. coli RRI transformed with pM220 has been deposited with ATCC under accession number 39853. Plasmid pM220 was digested with Eco RI and the 0.9 kb fragment comprising the TPI1 promoter was isolated by agarose gel electrophoresis end the ends were blunted with DNA polymerase I (Klenow fragment). Kinased Xba I linkers were added to the fragment, which was then digested with Bgl II and Xba I. This modified TPI1 promoter fragment was then ligated into the 3.4 kb Bgl II-Xba I vector fragment of pDR1107 to produce pZV118. Plasmid pDR1107 was constructed by subcloning the 900 bp Bgl II-Eco RI TPI1 promoter fragment of pM220 into pIC7 (Marsh, Erfle and Wykes, Gene 32: 481-485, 1984) to generate plasmid pDR1101. Plasmid pDR1101 was digested with Hind III and Sph I to isolate the 700 bp partial TPI1 promoter fragment. Plasmid pDR1100, comprising the 800 bp Xba I-Bam HI TPI1 terminator fragment of pM220 subcloned into pUC18, was cut with Hind III and Sph I. The 700 bp partial TPI1 promoter was ligated into the linearized pDR1100 to produce pDR1107.

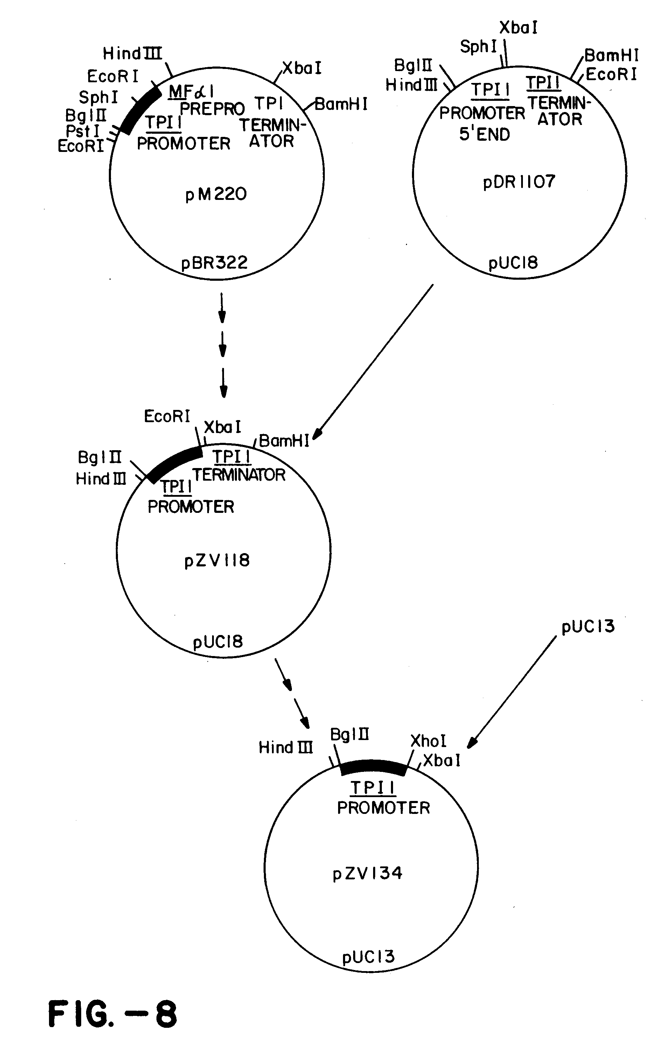

The Eco RI site at the 3' end of the TPI1 promoter in pZV118 was then destroyed. The plasmid was digested with Hind III and Eco RI and the 0.9 kb fragment was isolated and ligated to a synthetic linker constructed by annealing oligonucleotides ZC708 (^{5'}AATTGCTCGAGT^{3'}) and ZC709 (^{3'}CGAGCTCAGATC^{5'}). The linker addition eliminates the Eco RI site at the 3' terminus of the TPI1 promoter fragment and adds XhoI and Xba I sites. This fragment was then joined to Hind III-Xba I cut pUC13. The resultant plasmid was designated pZV134 (Figure 8).

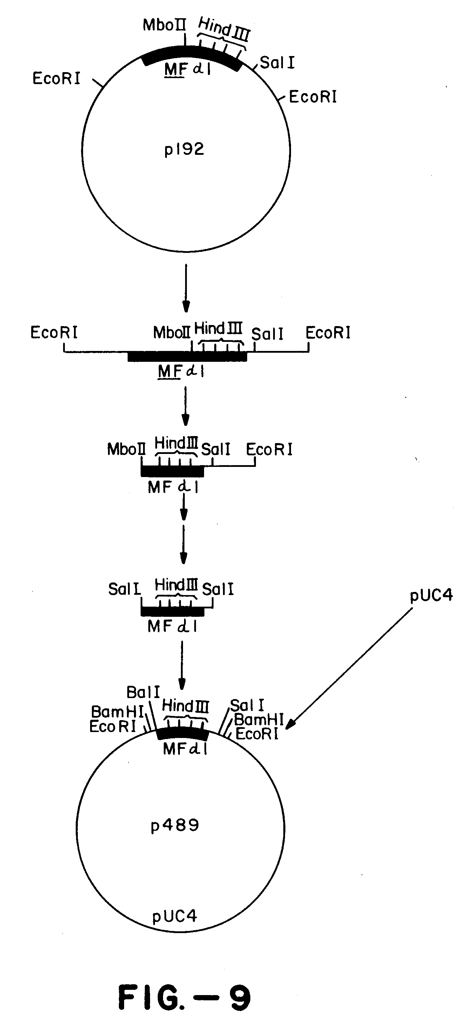

Cloning of the yeast mating pheromone α-factor (MFα1) gene has been described by Kurjan and Herskowitz (ibid). The gene was isolated in this laboratory in a similar manner from a yeast genomic library of partial Sau 3A fragments cloned into the Bam HI site of YEp13 (Nasmyth and Tatchell, Cell 19: 753-764, 1980). From this library, a plasmid was isolated which expressed α-factor in a diploid strain of yeast homozygous for the matα2-34 mutation (Manney, et al., J. Cell. Biol., 96: 1592, 1983). The clone contained an insert overlapping with the MFα1 gene characterized by Kurjan and Herskowitz. This plasmid, known as pZA2, was digested with Eco RI and the 1.7 kb fragment containing MFα1 was isolated and ligated into Eco RI cut pUC13. The resultant plasmid, designated p192, was cleaved with Eco RI and the resultant 1.7 kb MFα1 fragment was isolated and digested with Mbo II. The 550 bp Mbo II-Eco RI fragment was isolated and ligated to kinased Sal I linkers. The linkered fragment was cut with Sal I. The resulting 0.3 kb Sal I fragment was ligated into Sal I cut pUC4 (Vieira and Messing, Gene, 19: 259-268, 1982) to produce a plasmid designated p489 (Figure 9).

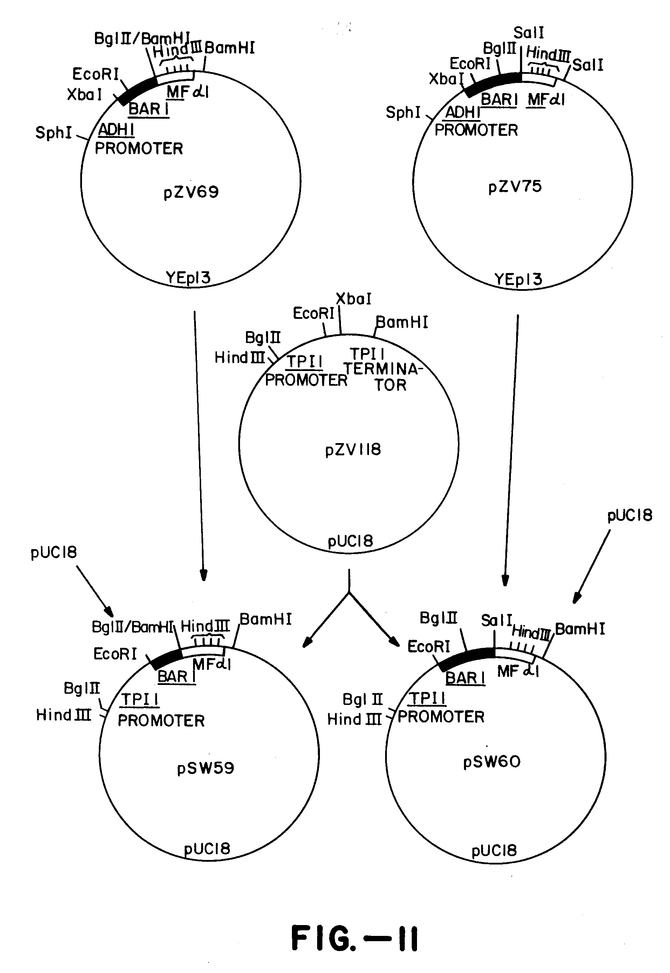

A gene fusion comprising portions of the BAR1 (114 codons) and MFα1 coding sequence was then constructed. Plasmid pZV24 (Example 2) was digested with Sph I and Bgl II and the 0.8 kb ADH1 promoter-BAR1 fragment was isolated. Plasmid p489 was cleaved with Bam HI and the 0.3 kb MFα1 fragment was isolated. These two fragments were joined in a three part ligation to Sph I+Bam HI cut YEp13. The resultant plasmid was designated pZV69 (Figure 11).

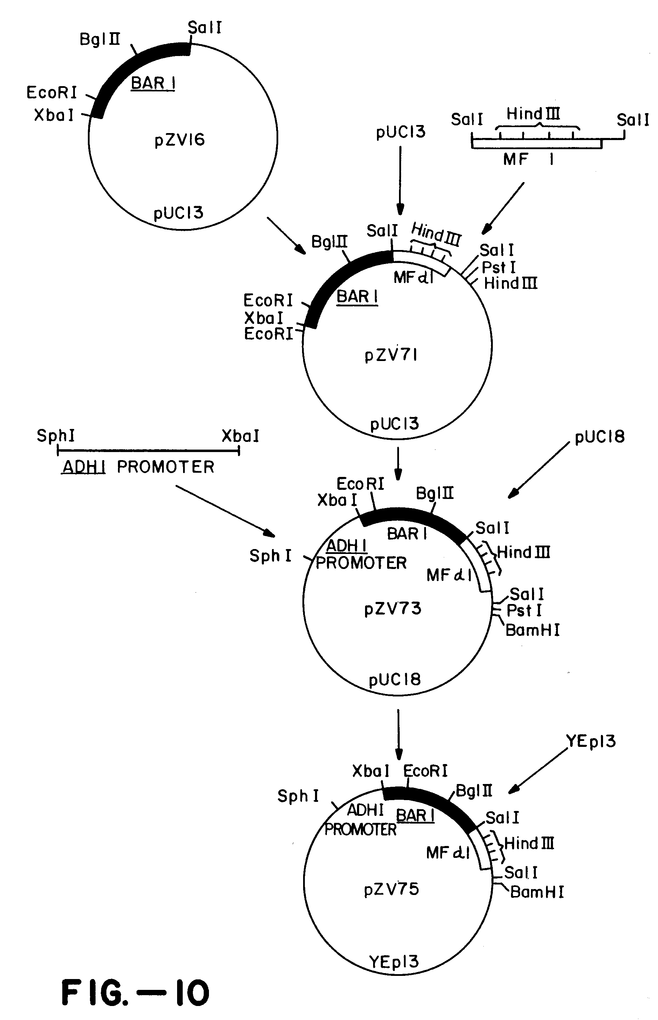

A second fusion gene encoding 25l amino acids of Barrier joined to a portion of the alpha-factor percursor was constructed. Plasmid pZV16, containing the 767 bp Xba I-Sal I BAR1 fragment from pZV9 (Example 1) ligated into Xba I+Sal I cut pUC13, was linearized by digestion with Sal I. This 4.0 kb fragment was joined with the 0.3 kb Sal I fragment from p489 encoding the four copies of α-factor. A plasmid having the BAR1-MFα1 fusion in the correct orientation was designated as pZV71. The BAR1-MFα1 fusion from pZV71 was then joined to the ADH1 promoter. Plasmid pZV71 was digested with Xba I and Pst I and the 1.07 kb fragment was isolated. The ADH1 promoter was isolated as a 0.42 kb Sph I-Xba I fragment from pZV24. These two fragments were joined, in a three part ligation, to Sph I+Pst I cut pUC18. The resulting plasmid, pZV73, was digested with Sph I and Bam HI and the 1.5 kb fragment comprising the expression unit was isolated and ligated into the Sph I+Bam HI cut YEp13 to form pZV75 (Figure 10).

For ease of manipulation, the BAR1-MFα1 fusion units from pZV69 and pZV75 were subcloned with the TPI1 promoter into pUC18 (Figure 11). Plasmid pZV69 was digested with Eco RI and Bam HI and the 0.55 kb fragment containing the fusion was isolated. The 0.9 kb TPI1 promoter fragment was isolated from pZV118 by digestion with Hind III and Eco RI. A three part ligation was done by using the .55 kb BAR1-MFα1 fragment, the 0.9 kb TPI1 promoter fragment and pUC19 cut with Hind III and Bam HI. The resultant plasmid was designated pSW59. Plasmid pZV75 was digested with Eco RI and Bam HI to isolate the 954 bp BAR1-MFα1 fusion fragment. This BAR1-MFα1 fragment was ligated in a three part ligation with the 0.9 kb Hind III+Eco RI TPI1 promoter fragment and pUC18 cut with Hind III and Bam HI to generate plasmid pSW60.

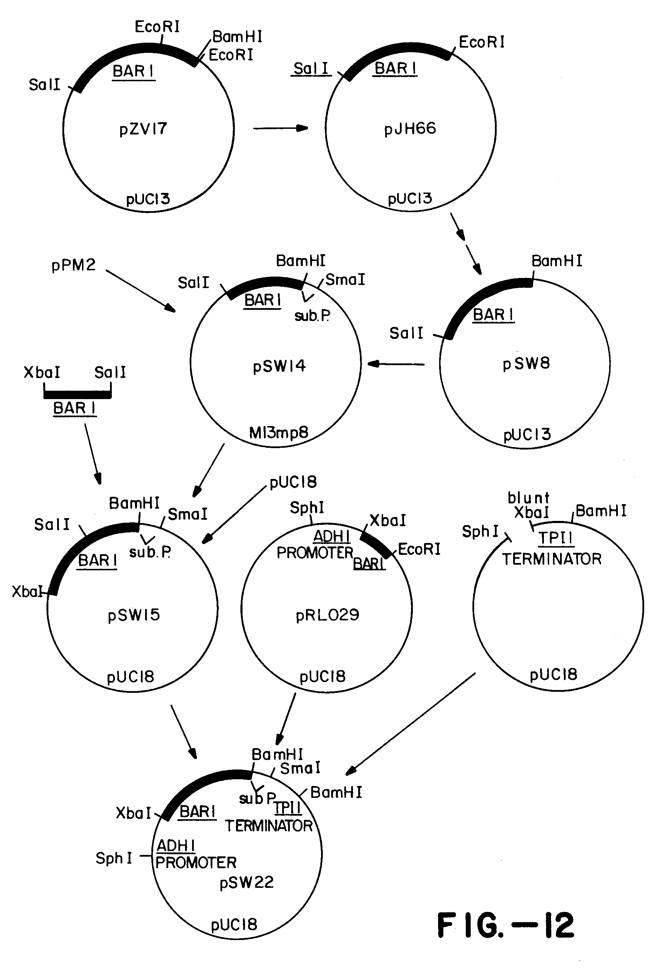

In construction of expression plasmids, the source of the 5' 116 bp of the BAR1 coding sequence was pSW22, which was constructed in the following manner (Figure 12). The BAR1 coding region found in pSW22 originated from pZV9. Plasmid pZV9 (Example 1) was cut with Sal I and Bam HI to isolate the 1.3 kb BAR1 fragment. This fragment was subcloned into pUC13 cut with Sal I and Bam HI to generate the plasmid designated pZV17. Plasmid pZV17 was digested with Eco RI to remove the 3'-most 0.5 kb of the BAR1 coding region. The vector-BAR1 fragment was religated to create the plasmid designated pJH66. Plasmid pJH66 was linearized with Eco RI and blunt-ended with Klenow fragment. Kinased Bam HI linkers (^{5'}CCGGATCCGG^{3'}) were added and excess linkers were removed by digestion with Bam HI before religation. The resultant plasmid, pSW8, was cut with Sal I and Bam HI to isolate the 824 bp fragment encoding amino acids 252 through 525 of BAR1. This BAR1 fragment was fused to a fragment encoding the C-terminal portion of substance P (Munro and Pelham, EMBO J., 3: 3087-3093, 1984). Plasmid pPM2, containing the synthetic oligonucleotide sequence encoding the dimer form of substance P in M13mp8, was obtained from Munro and Pelham. Plasmid pPM2 was linearized by digestion with Bam HI and Sal I and ligated with the 824 bp BAR1 fragment. The resultant plasmid pSW14 was digested with Sal I and Sma I to isolate the 871 bp BAR1-substance P fragment. Plasmid pZV16 (Figure 10) was cut with Xba I and Sal I to isolate the 767 bp 5' coding sequence of BAR1. This fragment was ligated with the 871 bp BAR1-substance P fragment in a three part ligation with pUC18 cut with Xba I and Sma I. The resultant plasmid was designated pSW15. Plasmid pSW15 was digested with Xba I and Sma I to isolate the 1.64 kb BAR1-substance P fragment. The ADH1 promoter was obtained from pRL029, comprising the 0.54 kb Sph I-Eco RI fragment containing the ADH1 promoter and 116 bp of the BAR1 5' coding region from pZV24 in pUC18. Plasmid pRL029 was digested with Sph I and Xba I to isolate the 0.42 kb ADH1 promoter fragment. The TPI1 terminator (Alber and Kawasaki, J. Mol. Appl. Gen. 1: 419-434, 1982) was provided as a 0.7 kb Xba I+Eco RI fragment in pUC18. The linearized fragment containing the TPI1 terminator and pUC18 with a Klenow filled Xba I end and an Sph I end was ligated with the 0.42 kb ADH1 promoter fragment and the 1.64 kb BAR1-substance P fragment in a three part ligation to produce plasmid pSW22.

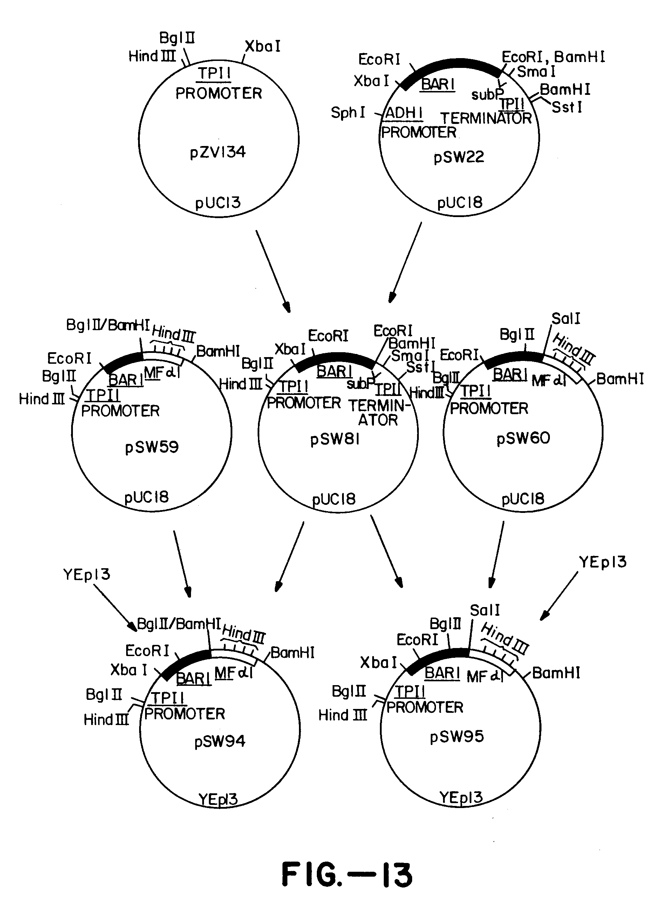

Plasmid pSW94 was then constructed (Figure 13). The 2.3 kb fragment containing the BAR1-substance P fusion and the TPI1 terminator was isolated from plasmid pSW22 as an Xba I-Sst I fragment. The Hind III-Xba I TPI1 promoter fragment isolated from pZV134 was joined to the BAR1-substance P-TPI1 terminator fragment in a three part ligation with Hind III+SstI cut pUC18. The resultant plasmid, pSW81, was cleaved with Hind III and Eco RI to isolate the 1.02 kb fragment containing the TPI1 promoter and the 5' 116 bp of BAR1. Plasmid pSW59 was cut with Eco RI and Bam HI to isolate the 0.55 kb BAR1-MFα1 fusion fragment. This fragment was then ligated in a three part ligation with the TPI1 promoter-BAR1 fragment from pSW81 and YEp13 linearized with Hind III and Bam HI resulting in plasmid pSW94.

The construction of pSW95 is illustrated in Figure 13. Plasmid pSW60 was cut with Eco RI and Bam HI to isolate the 954 bp BAR1-MFα1 fusion fragment. Plasmid pSW81 was cut with Hind III and Eco RI to isolate the 1.02 kb TPI1 promoter-BAR1 fragment which was joined with the BAR1-MFα1 fusion fragment in a three part ligation into Hind III+Bam HI cut YEp13. The resultant plasmid was designated pSW95.

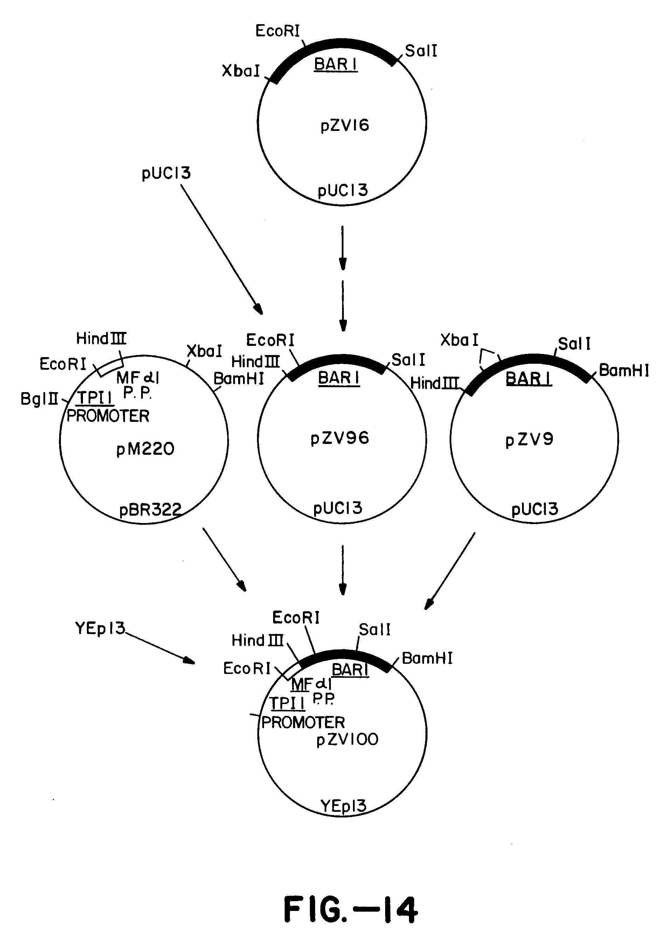

TPI1 promoter-BAR1-MFα1 fusion constructs containing only one copy of α-factor originated from BAR1-MFα1 fusions (encoding four copies of α-factor) that contained the TPI1 promoter and MFα1 prepro sequence (see Figures 14, 15, and 16). Plasmid pZV16 was digested with Eco RI and Sal I. The isolated 651 bp BAR1 fragment was ligated with a kinased Hind III-Eco RI BAR1 specific adaptor (produced by annealing oligonucleotides ZC566:
⁵'AGCTTTAACAAACGATGGCACTGGTCACTTAG^{3'} and ZC567:
^{5'}AATTCTAAGTGACCAGTGCCATCGTTTGTTAA^{3'}) into pUC13 cut with Hind III and Sal I. The resultant plasmid, pZV96, was digested with Hind III and Sal I to isolate the 684 bp BAR1 fragment. Plasmid pM220 provided the TPI1 promoter fused to the MFα1 prepro sequence. Plasmid pM220 was digested with Bgl II and Hind III to isolate the 1.2 kb TPI1 promoter-MFα1 prepro fragment. The 3' portion of the BAR1 coding region was obtained by cutting pZV9 with Sal I and Bam HI to isolate the 1.3 kb BAR1 fragment. The 684 bp Hind III-Sal I BAR1 fragment, the 1.2 kb Bgl II-Hind III TPI1 promoter-MFα1 prepro fragment and the 1.3 kb Sal I-Bam HI BAR1 fragment were joined with YEp13 linearized with Bam HI in a four part ligation. The construct with the desired orientation of promoter and MFα1-BAR1 fusion was designated pZV100 (Figure 14).

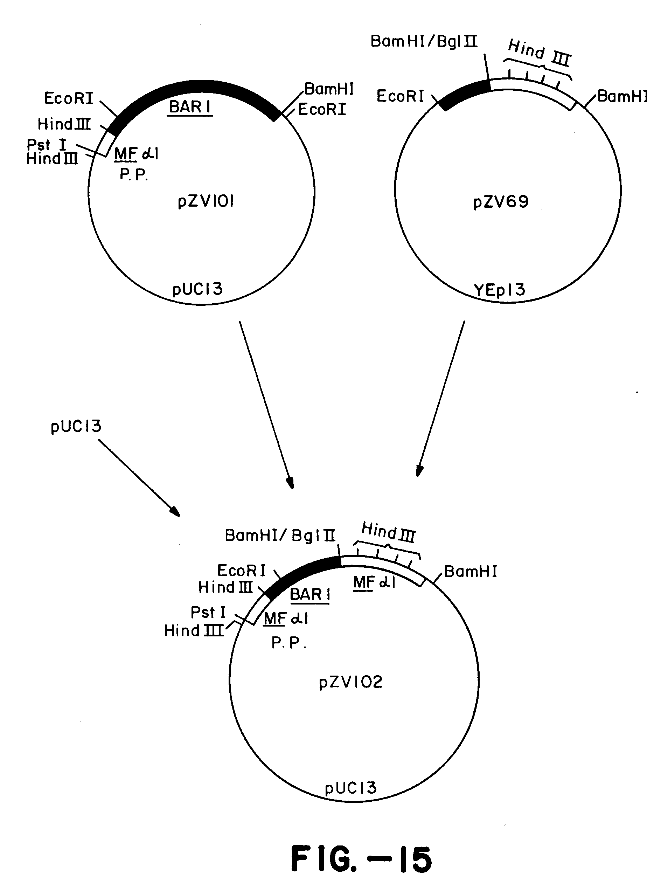

For ease of manipulation, the truncated MFα1 prepro sequence-BAR1 fusion fragment from pZV100 was subcloned into pUC13 as a 1.6 kb Pst I-Bam HI fragment. The resultant plasmid, pZV101, was cleaved with Pst I and Eco RI to isolate the 270 bp MFα1 prepro-BAR1 fragment. Plasmid pZV69 was digested with Eco RI and Bam HI to isolate the 0.55 kb BAR1-MFα1 fusion fragment (encoding four copies of α-factor). This fragment and the 270 bp MFα1 prepro-BAR1 fragment were ligated in a three part ligation into pUC13 cut with Pst I and Bam HI. The resultant plasmid was designated pZV102 (Figure 15).

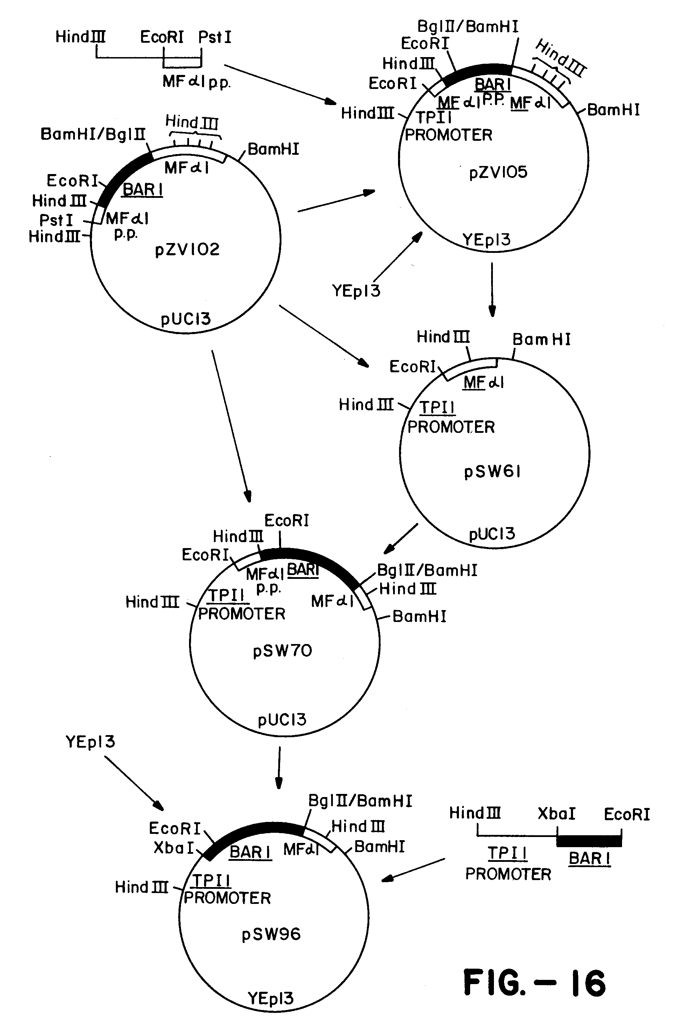

An expression unit comprising the TPI1 promoter, a portion of BAR1, and a single copy of the α-factor coding sequence was then constructed (Figure 16). Plasmid pZV102 was cut with Pst I and Bam HI to isolate the 0.82 kb MFα1 prepro-BAR1 fragment. A 1 kb Hind III-Pst I fragment comprising the TPI1 promoter and the truncated MFα1 prepro sequence from pM220 was joined to the 0.82 kb MFα1 prepro-BAR1 fragment isolated from pZV102 in a three part ligation with YEp13 cut with Hind III and Bam HI. The resultant plasmid was designated pZV105. Plasmid pZV105 was cleaved with Hind III to isolate the 1.2 kb TPI1 promoter-MFα1 prepro fragment. Plasmid pZV102 was digested with Hind III to isolate the vector fragment containing the terminal α-factor copy. This 2.8 kb vector-MFα1 fragment was ligated to the 1.2 kb TPI1 promoter-MFα1 prepro fragment. The plasmid with the correct orientation and a single copy of MFα1 coding sequence was designated pSW61. Plasmid pSW61 was linearized by a partial digestion with Hind III. Plasmid pZV102 was digested with Hind III to isolate the 0.3 kb BAR1-MFα1 fragment. This fragment was ligated into the linearized pSW61. The plasmid with the insert in the correct orientation at the Hind III site 264 bp 3' to the MFα1 start codon was designated pSW70. Plasmid pSW70 was cleaved with Eco RI and Bam HI to isolate the 361 bp BAR1-MFα1 fragment. Plasmid pSW81 (Figure 13) was digested with Hind III and Eco RI to isolate the 1.02 kb TPI1 promoter-BAR1 fragment. This fragment was joined to the BAR1-MFα1 fragment in a three part ligation with YEp13 linearized with Hind III and Bam HI. The resultant plasmid, pSW96, contains the TPI1 promoter and 356 bp of the 5' coding sequence of BAR1 fused to one copy of the α-factor coding sequence.

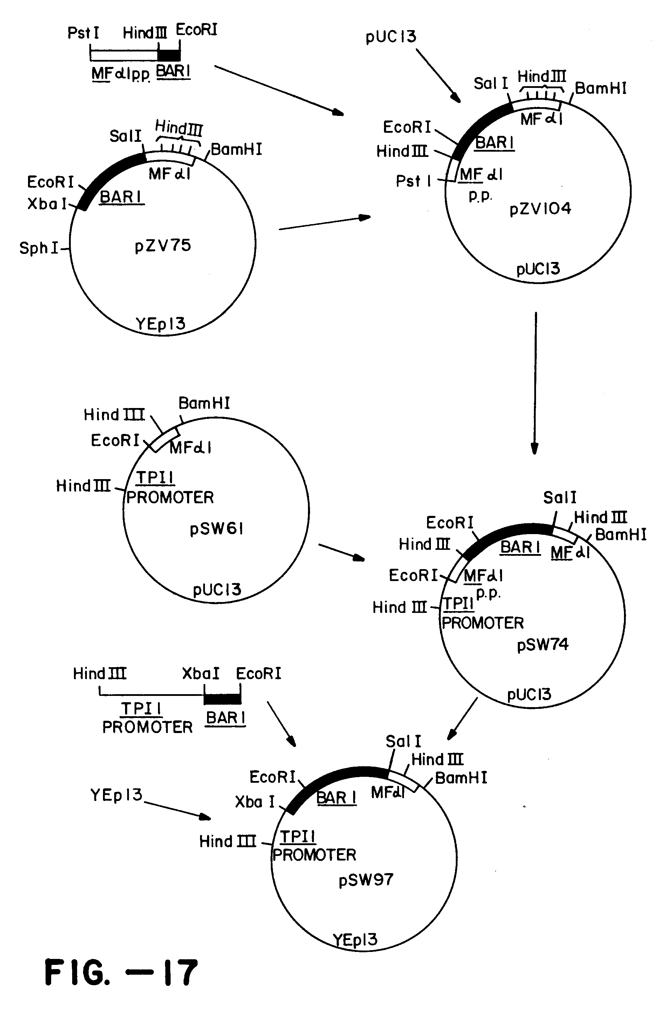

The second BAR1-MFα1 construct containing 767 bp of BAR1 fused to one copy of the MFα1 coding sequence was made using pZV75 as the source of the BAR1 fragment (Figure 17). Plasmid pZV75 was digested with Eco RI and Bam HI to isolate the 954 bp BAR1-MFα1 fragment. Plasmid pZV101, containing the MFα1 prepro sequence fused to BAR1, was cut with Pst I and Eco RI to isolate the 0.27 kb MFα1 prepro-BAR1 fragment. This fragment was joined to the 954 bp BAR1-MFα1 fragment in a three part ligation with pUC13 linearized with Pst I and Bam HI. The resultant plasmid, pZV104, was cleaved with Hind III to isolate the 0.70 kb BAR1-MFα1 fragment. This fragment was ligated to pSW61 which was linearized by partial digestion with Hind III. The plasmid with the insert in the correct orientation at the Hind III site 264 bp 3' to the start codon of MFα1 was designated pSW74. Plasmid pSW74 was cut with Eco RI and Bam HI to isolate the 738 bp BAR1-MFα1 fragment. Plasmid pSW81 was cut with Hind III and Eco RI to isolate the 1.02 kb TPI1 promoter-BAR1 fragment. This fragment was joined to the 738 bp BAR1-MFα1 fragment in a three part ligation with Hind III+Bam HI cut YEp13. The resultant plasmid, pSW97, contains the TPI1 promoter and 767 bp of the 5' and of BAR1 fused to the single copy of the ^{α}-factor coding sequence.

The a/α diploid S. cerevisiae strain XP733 (MATa leu2-3 leu2-112 bar1-1 gal2/MATα leu2-3 leu2-112 bar1-1 gal2) was transformed with plasmids pSW73, pSW94 and pSW95. Plasmid pSW73 comprises the TPI1 promoter, MFα1 signal peptide and prepro sequence and the coding region for the four copies of α-factor in YEp13. The transformants were spotted onto a lawn of S. cerevisiae RC629 cells in a soft agar overlay over a plate of selective media and incubated overnight at 30°C. A comparison of halo sizes using pSW73 and the control shows that pSW94 exports approximately 15% as much α-factor as pSW73.

The constructs containing BAR1 fused to one copy of the MFα1 coding sequence were assayed for export of α-factor in the same manner. Plasmid pSW67, comprising the TPI1 promoter, MFα1 signal peptide, prepro and the coding region for one copy of α-factor in YEp13 was used as a control for plasmids pSW96 and pSW97. A comparison between halo sizes indicated that pSW96 directs secretion of approximately 30 - 40% as much α-factor as pSW67 and pSW97 directs secretion of approximately 10 - 15% as much α-factor as pSW67.

### EXAMPLE 5

### MUTATION OF THE BAR1 SIGNAL PEPTIDE CLEAVAGE SITE

As described above, it has been found that altering the signal peptide cleavage site of the Barrier precursor could be expected to facilitate processing and export of Barrier-containing fusion proteins through the KEX2 pathway. Potential cleavage sites are between amino acids 23 and 24 and between amino acids 24 and 25. Thus, the DNA sequence coding for the BAR1 primary translation product was mutated to encode a proline residue at position 25. Plasmids pSW98 and pSW99 are YEp13-based plasmids comprising the S. cerevisiae TPI1 promoter, a 355 bp or 767 bp fragment of the BAR1 gene, including the mutated signal peptide cleavage site, and one copy of the α-factor coding sequence.

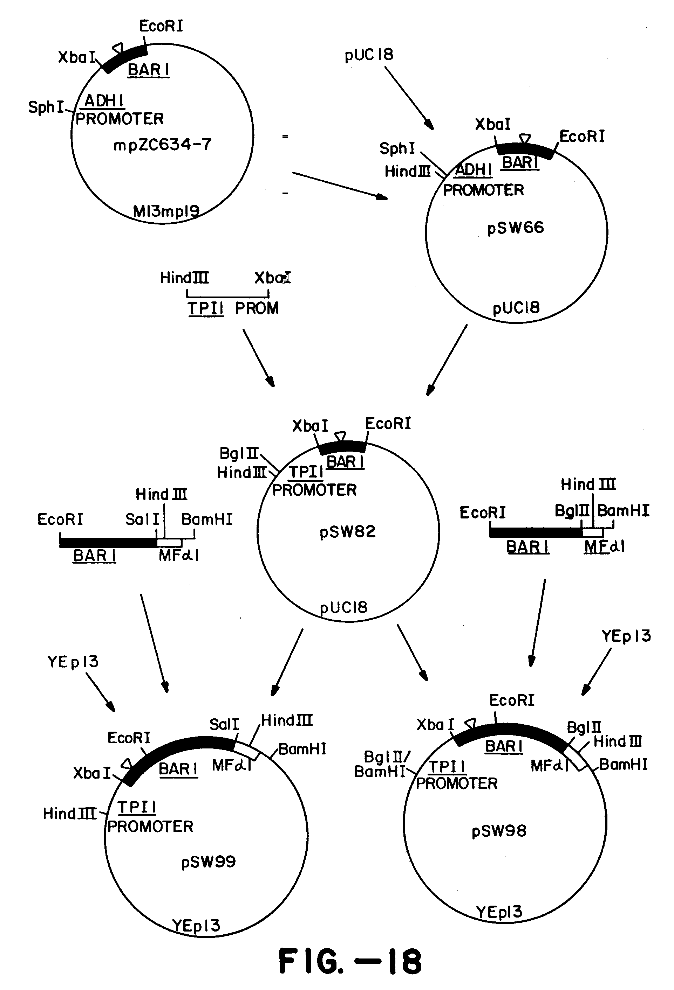

The signal peptide mutation was introduced by standard in vitro mutagenesis methods (Zoller et al., Manual for Advanced Techniques in Molecular Cloning Course, Cold Spring Harbor Laboratory, 1983) using a phage M13 template and a synthetic mutagenic oligonucleotide (sequence ^{5'}ATTACTGCTCCTACAAACGAT^{3'}). The phage template pSW54 was constructed by ligating the 0.54 kb Sph I-Eco RI fragment of pSW22 with Sph I-Eco RI digested M13mp19. Following in vitro mutagenesis, potentially mutagenized plaques were screened by plaque hybridization with ³²P-labeled mutagenic oligonucleotide and were sequenced to confirm the presence of the mutation. The replicative form of one of the confirmed mutagenized phage, mZC634-7, was digested with Sph I and Eco RI and the 0.54 kb fragment was isolated and ligated with Sph I+Eco RI cut pUC18. The resulting plasmid, pSW66 (Figure 18), was digested with Hind III and Xba I to remove the ADH1 promoter, and the fragment comprising the vector and BAR1 sequences was ligated with the 0.9 kb Hind III-Xba I TPI1 promoter fragment of pZV134. This plasmid, with the TPI1 promoter and 119 bp of the 5' end of BAR1 including the signal peptide cleavage mutation, was designated pSW82.

Referring to Figure 18, plasmid pSW82 was digested with Hind III and Eco RI and with Bgl II and Eco RI and the resulting 1.02 kb fragments were isolated. The Hind III-Eco RI fragment of pSW82 was ligated with the Eco RI-Bam HI fragment of pSW74 and Hind III+Bam HI digested YEp13 to form pSW99. The Bgl II-Eco RI fragment of pSW82 was ligated with the 0.30 kb Eco RI-Bam HI fragment of pSW70 and Bam HI digested YEp13, to form pSW98. Plasmid pSW98 includes the TPI1 promoter, 355 bp of the 5' end of the mutagenized BAR1 sequence and a single copy of the α-factor coding sequence. Plasmid pSW99 contains the identical expression unit except for having 767 bp of the mutagenized BAR1 sequence.

An analysis by the halo assay showed that the cleavage site mutation enhanced α-factor export when using the plasmids encoding one copy of α-factor. Transformants containing pSW98 exported about 50% more α-factor than those containing pSW96, the wild-type control.

## Claims

1. A DNA construct comprising the following elements, operably linked in a 5' to 3' orientation as follows:
a transcriptional promoter;
a portion of the Saccharomyces cerevisiae BAR1 gene: according to Fig. 1 encoding a polypeptide consisting essentially of between 24 and 405 amino acids encoded by the amino-terminal sequence of the BAR1 gene starting with the initiation methionine; and
at least one structural gene foreign to a selected host.

2. A construct according to Claim 1 wherein said construct further encodes a KEX2 processing site between the portion of the BAR1 gene and the structural gene.

3. A construct according to Claim 2 wherein said signal peptide coding sequence of said BAR1 gene is altered by site-specific mutagenesis to reduce the efficiency of signal peptidase cleavage of the fusion polypeptide or protein.

4. A construct according to Claim 1 wherein said promoter is a yeast glycolytic pathway gene promoter.

5. A construct according to Claim 1 wherein said promoter is selected from the group consisting of the S. cerevisiae BAR1 promoter, S. cerevisiae alcohol dehydrogenase I promoter, and Schizosaccharomyces pombe alcohol dehydrogenase promoter.

6. A construct according to Claim 1 further comprising the transcription terminator region of the Saccharomyces cerevisiae triose phosphate isomerase gene.

7. A construct according to Claim 1 wherein said portion of said BAR1 gene further comprises the 680 base pair sequence of the 5'-untranslated region adjacent to the translation initiation site.

8. A DNA construct according to Claim 1 wherein said polypeptide consists essentially of 178 amino acids encoded by the amino-terminal sequence of the BAR1 gene starting with the initiation methionine.

9. A DNA construct according to Claim 1 wherein said polypeptide consists essentially of 251 amino acids encoded by the amino-terminal sequence of the BAR1 gene starting with the initiation methionine.

10. A transformed cell containing a construct according to any of the Claims 1-9.

11. A transformed cell according to Claim 10 wherein said cell is a fungal cell.

12. A cell according to Claim 11 wherein said fungus is Saccharomyces cerevisiae.

13. A cell according to Claim 11 wherein said fungus is Schizosaccharomyces pombe.

14. A cell according to Claim 11 wherein said fungus is Aspergillus or Neurospora.

15. A method for producing and secreting a heterologous protein from a transformed cell comprising the steps of:
(a) transforming a host cell with a DNA construct comprising the following elements, operably linked in a 5' to 3' orientation as follows:
a transcriptional promoter;
a portion of the S. cerevisiae BAR1 gene: according to Fig. 1 encoding a polypeptide consisting essentially of between 24 and 405 amino acids encoded by the amino-terminal sequence of the BAR1 gene starting with the initiation methionine; and
a structural gene encoding said heterologous protein; and
(b) growing said cell from step (a) under growth conditions suitable for the production of said protein encoded by said DNA construct.

16. A method according to Claim 15 wherein said cell is a fungal cell.

17. A method according to Claim 16 wherein said fungus is Saccharomyces cerevisiae.

18. A method according to Claim 16 wherein said fungus is Schizosaccharomyces pombe.

19. A cell according to Claim 16 wherein said fungus is Aspergillus or Neurospora.

20. A method according to Claim 15 wherein said fusion protein comprises a site cleaved by the product of the S. cerevisiae KEX2 gene.

21. A method according to Claim 20 wherein said signal peptide coding sequence of said BAR1 gene is altered by site-specific mutagenesis to reduce the efficiency of signal peptidase cleavage of the polypeptide or protein.

22. A method according to Claim 15 wherein said promoter is selected from the group consisting of the S. cerevisiae BAR1 promoter, S. cerevisiae alcohol dehydrogenase I promoter, and Schizosaccharomyces pombe alcohol dehydrogenase promoter.

23. A method according to Claim 15 wherein said promoter is a yeast glycolytic pathway gene promoter.

24. A method according to Claim 15 wherein said heterologous protein is exported from said cell.

25. A method according to Claim 15 wherein said protein comprises proinsulin or insulin.

26. A method according to Claim 15 wherein said polypeptide consists essentially of 178 amino acids encoded by the amino-terminal sequence of the BAR1 gene starting with the initiation methionine.

27. A method according to Claim 15 wherein said polypeptide consists essentially of 251 amino acids encoded by the amino-terminal sequence of the BAR1 gene starting with the initiation methionine.

## Patentansprüche

1. DNA-Gebilde, das die nachfolgend aufgeführten Komponenten umfaßt, die miteinander in einer 5' - 3'-Orientierung funktionell wie folgt verknüpft sind:
ein Transkriptionspromotor,
ein Teil des Saccharomyces cerevisiae-BAR1-Gens nach Fig. 1, das ein Polypeptid kodiert, das im wesentlichen aus 24 bis 405 Aminosäuren besteht, die durch die Aminoterminalsequenz des mit dem Initiationsmethionin beginnenden BAR1-Gens kodiert wurden und
mindestens ein für einen ausgewählten Wirt fremdes Strukturgen.

2. Gebilde nach Anspruch 1, bei dem dieses noch einen KEX2-Processing-Site zwischen dem Teil des BAR1-Gens und dem Strukturgen kodiert.

3. Gebilde nach Anspruch 2, bei dem die Signalpeptid-Kodierungssequenz des BAR1-Gens zur Verminderung der Wirksamkeit der Signalpeptidase-Spaltung des Fusionspolypeptids oder -proteins durch sitespezifische Mutagenese abgeändert ist.

4. Gebilde nach Anspruch 1, bei dem der Promotor ein Hefe-Glycolyse-Genpromotor ist.

5. Gebilde nach Anspruch 1, bei dem der Promotor ausgewählt ist aus der Gruppe, bestehend aus S. cerevisiae BAR1-Promotor, S. cerevisiae-Alkoholdehydrogenase I-Promotor und Schizosaccharomyces pombe-Alkoholdehydrogenase-Promotor.

6. Gebilde nach Anspruch 1, das außerdem noch den Transkriptionsterminatorbereich des Saccharomyces cerevisiae-Triosephosphatisomerase-Gens umfaßt.

7. Gebilde nach Anspruch 1, bei dem der Teil des BAR1-Gens auch noch die 680 bp-Sequenz des an den Translationsinitiations-Site angrenzenden 5'-nichtübersetzten Bereichs umfaßt.

8. DNA-Gebilde nach Anspruch 1, bei dem das Polypeptid im wesentlichen aus 178 Aminosäuren besteht, die durch die Aminoterminalsequenz des mit dem Initiationsmethionin beginnenden BAR1-Gens kodiert wurden.

9. DNA-Gebilde nach Anspruch 1, bei dem das Polypeptid im wesentlichen aus 251 Aminosäuren besteht, die durch die Aminoterminalsequenz des mit dem Initiationsmethionin beginnenden BAR1-Gens kodiert wurden.

10. Eine ein Gebilde nach einem der Ansprüche 1 bis 9 enthaltende transformierte Zelle.

11. Transformierte Zelle nach Anspruch 10, wobei diese eine Pilzzelle darstellt.

12. Zelle nach Anspruch 11, wobei der Pilz Saccharomyces cerevisiae ist.

13. Zelle nach Anspruch 11, wobei der Pilz Schizosaccharomyces pombe ist.

14. Zelle nach Anspruch 11, wobei der Pilz Aspergillus oder Neurospora ist.

15. Verfahren zur Herstellung und Sekretion eines heterologen Proteins aus einer transformierten Zelle, das folgende Stufen umfaßt:
(a) Transformation einer Wirtszelle mit einem DNA-Gebilde, das die nachfolgend aufgeführten Komponenten umfaßt, die miteinander in einer 5' - 3'-Orientierung operabel wie folgt verknüpft sind:
einen Transkriptionspromotor,
einen Teil des S. cerevisiae BAR1-Gens nach Fig. 1, das ein Polypeptid kodiert, das im wesentlichen aus 24 bis 405 Aminosäuren besteht, die durch die Aminoterminalsequenz des mit dem Initiationsmethionin beginnenden BAR1-Gens kodiert wurden; und
ein Strukturgen, das das heterologe Protein kodiert; und
(b) Züchtung der Zelle aus Stufe (a) unter für die Produktion des durch das DNA-Gebilde kodierten Proteins geeigneten Wachstumsbedingungen.

16. Verfahren nach Anspruch 15, worin die Zelle eine Pilzzelle darstellt.

17. Verfahren nach Anspruch 16, worin der Pilz Saccharomyces cerevisiae ist.

18. Verfahren nach Anspruch 16, worin der Pilz Schizosaccharomyces pombe ist.

19. Verfahren nach Anspruch 16, worin der Pilz Aspergillus oder Neurospora ist.

20. Verfahren nach Anspruch 15, worin das Fusionsprotein einen durch das Produkt des S. cerevisiae KEX2-Gens gespaltenen Site umfaßt.

21. Verfahren nach Anspruch 20, worin die Signalpeptid-Kodierungssequenz des BAR1-Gens zur Verminderung der Wirksamkeit der Signalpeptidase-Spaltung des Polypeptids oder Proteins durch sitespezifische Mutagenese abgeändert ist.

22. Verfahren nach Anspruch 15, worin der Promotor ausgewählt ist aus der Gruppe, bestehend aus S. cerevisiae BAR1-Promotor, S. cerevisiae-Alkoholdehydrogenase I-Promotor und Schizosaccharomyces pombe-Alkoholdehydrogenase-Promotor.

23. Verfahren nach Anspruch 15, worin der Promotor ein Hefe-Glycolyse-Genpromotor ist.

24. Verfahren nach Anspruch 15, worin das heterologe Protein aus der Zelle transportiert wird.

25. Verfahren nach Anspruch 15, worin das Protein Proinsulin oder Insulin umfaßt.

26. Verfahren nach Anspruch 15, worin das Polypeptid im wesentlichen aus 178 Aminosäuren besteht, die durch die Aminoterminalsequenz des mit dem Initiationsmethionin beginnenden BAR1-Gens kodiert wurden.

27. Verfahren nach Anspruch 15, worin das Polypeptid im wesentlichen aus 251 Aminosäuren besteht, die durch die Aminoterminalsequenz des mit dem Initiationsmethionin beginnenden BAR1-Gens kodiert wurden.

## Revendications

1. Structure d'ADN comprenant les éléments suivants, liés de manière fonctionnelle selon une orientation 5' à 3', comme suit :
- un promoteur de transcription
- une portion du gène du Saccharomyces cerevisiae BAR1 selon la Figure 1 codant un polypeptide essentiellement constitué de 24 à 405 acides aminés codés par la séquence amino-terminale du gène BAR1 commençant avec la méthionine initiale ; et au moins un gène structural étranger à un hôte sélectionné.

2. Structure selon la Revendication 1, dans laquelle ladite structure code additionnellement un site de traitement KEX2 entre la portion du gène BAR1 et le gène structural.

3. Structure selon la Revendication 2, dans laquelle ladite séquence de codage du peptide signal dudit gène BAR1 est modifiée par mutagénèse spécifique de site afin de réduire l'efficacité de la coupure de la peptidase signal de la protéine ou du polypeptide de fusion.

4. Structure selon la Revendication 1, dans laquelle ledit promoteur est un promoteur de gène des voies glycolytiques de la levure.

5. Structure selon la Revendication 1, dans laquelle ledit promoteur est choisi dans le groupe constitué par le promoteur S. cerevisiae BAR1, le promoteur S. cerevisiae alcool déhydrogénase I et le promoteur Schizosaccharomyces pombe alcool déhydrogénase.

6. Structure selon la Revendication 1, comprenant la région terminale de transcription du gène de la triose phosphate isomérase du Saccharomyces cerevisiae.

7. Structure selon la Revendication 1, dans laquelle ladite portion dudit gène BAR1 comprend additionnellement la séquence de la paire de base 680 de la région 5' non translatée adjacente au site de début de translation.

8. Structure d'ADN selon la Revendication 1, dans laquelle ledit polypeptide est essentiellement constitué de 178 acides aminés codés par la séquence amino-terminale du gène BAR1 débutant avec la méthionine initiale.

9. Structure d'ADN selon la Revendication 1, dans laquelle ledit polypeptide est essentiellement constitué de 251 acides aminés codés par la séquence amino-terminale du gène BAR1 débutant avec la méthionine initiale.

10. Cellule transformée contenant une structure selon l'une quelconque des Revendications 1 à 9.

11. Cellule transformée selon la Revendication 10, dans laquelle ladite cellule est une cellule fongique.

12. Cellule selon la Revendication 11, dans laquelle ledit champignon est le Saccharomyces cerevisiae.

13. Cellule selon la Revendication 11, dans laquelle ledit champignon est le Schizosaccharomyces pombe.

14. Cellule selon la Revendication 11, dans laquelle ledit champignon est l'Aspergillus ou le Neurospora.

15. Procédé de production et de sécrétion d'une protéine hétérologue à partir d'une cellule transformée comprenant les étapes suivantes :
(a) transformation d'une cellule hôte avec une structure d'ADN comprenant les éléments suivants, liés de manière fonctionnelle selon une orientation 5' à 3', comme suit :
- un promoteur de transcription
- une portion du gène du S. cerevisiae BAR1 selon la Figure 1 codant un polypeptide essentiellement constitué de 24 à 405 acides aminés codés par la séquence amino-terminale du gène BAR1 commençant avec la méthionine initiale ; et un gène structural codant ladite protéine hétérologue ; et
(b) culture de ladite cellule à partir de l'étape (a) dans des conditions de culture propres à la production de ladite protéine codée par ladite structure d'ADN.

16. Procédé selon la Revendication 15 dans lequel ladite cellule est une cellule fongique.

17. Procédé selon la Revendication 16 dans lequel ledit champignon est le Saccharomyces cerevisiae.

18. Procédé selon la Revendication 16, dans lequel ledit champignon est le Schizosaccharomyces pombe.

19. Procédé selon la Revendication 16, dans lequel ledit champignon est l'Aspergillus ou le Neurospora.

20. Procédé selon la Revendication 15, dans lequel ladite protéine de fusion comprend un site coupé par le produit du gène KEX2 de la S. cerevisiae.

21. Procédé selon la Revendication 20, dans lequel ladite séquence de codage du peptide signal dudit gène BAR1 est modifiée par mutagénèse spécifique de site afin de réduire l'efficacité de la coupure de la peptidase signal du polypeptide ou de la protéine.

22. Procédé selon la Revendication 15, dans lequel ledit promoteur est choisi dans le groupe constitué par le promoteur S. cerevisiae BAR1, le promoteur S. cerevisiae alcool déhydrogénase I et le promoteur Schizosaccharomyces pombe alcool déhydrogénase.

23. Procédé selon la Revendication 15, dans lequel ledit promoteur est un promoteur de gène des voies glycolytiques de la levure.

24. Procédé selon la Revendication 15, dans lequel ladite protéine hétérologue est exportée de ladite cellule.

25. Procédé selon la Revendication 15, dans lequel ladite protéine comprend la proinsuline ou l'insuline.

26. Procédé selon la Revendication 15, dans lequel ledit polypeptide est essentiellement constitué de 178 acides aminés codés par la séquence amino-terminale du gène BAR1 débutant avec la méthionine initiale.

27. Procédé selon la Revendication 15, dans lequel ledit polypeptide est essentiellement constitué de 251 acides aminés codés par la séquence amino-terminale du gène BAR1 débutant avec la méthionine initiale.
